(19) Europäisches Patentamt
European Patent Office
Office européen des brevets

(11) EP 2 757 496 A1

(12) EUROPEAN PATENT APPLICATION

(43) Date of publication:
23.07.2014 Bulletin 2014/30

(51) Int Cl.:
*G06F 19/12* *(2011.01)* *G06F 19/20* *(2011.01)*

(21) Application number: 13151728.6

(22) Date of filing: 17.01.2013

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicants:
- Fondazione Edmund Mach
  38010 San Michele all'Adige (IT)
- Universita degli Studi di Trento
  38122 Trento (IT)

(72) Inventors:
- Moser, Claudio
  38122 Trento (IT)
- Coller, M Emanuela
  38030 Rovere' Della Luna (TN) (IT)
- Malacarne, M Giulia
  38121 Montevaccino (TN) (IT)
- Blanzieri, Enrico
  38100 Trento (IT)

(74) Representative: Lahrtz, Fritz
Isenbruck Bösl Hörschler LLP
Patentanwälte
Postfach 86 06 80
81635 München (DE)

# (54) Systems and methods for determining suitable entities for expanding established causal molecular biological networks and for determining significant causal relationships between entities of established causal molecular biological networks and candidate entities

(57) In a first aspect, there is provided a method for determining suitable entities for expanding an established causal molecular biological network, said method comprising: Providing an established causal molecular biological network, a candidate set of entities and abundance data thereof; Generating at least one intermediate expansion of said established causal molecular biological network with a defined part of the candidate set of entities by use of a computer program and by using the abundance data; Determining a measure of interrelationship for every entity of the candidate set of entities with respect to at least some of the entities of the established causal molecular biological network; Selecting entities out of the candidate set of entities based on the determined measure of interrelationship.

According to a second aspect, there is provided a method for determining significant causal relationships between entities of an established causal molecular biological network and candidate entities.

There are also provided a computer program and an apparatus, suitable for implementing the methods.

Figure 2

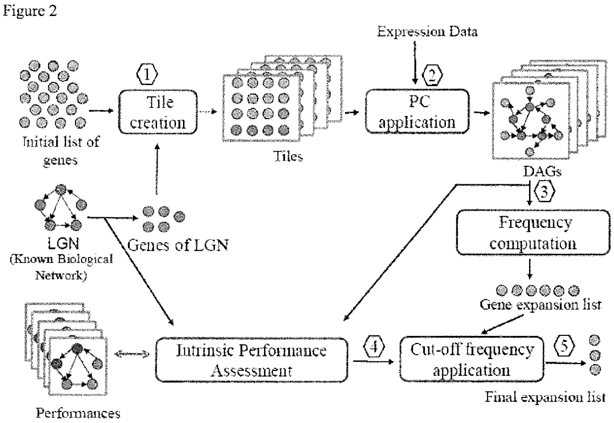


EP 2 757 496 A1

## Description

BACKGROUND

The invention relates to systems and methods for determining suitable entities for expanding established causal molecular biological networks and for determining significant causal relationships between entities of established causal molecular biological networks and candidate entities.

To understand the function of a cell or even an organism, it is essential to decipher how the constitutive entities are interacting. This is the main goal of systems biology which still holds young the promise to transform biology and medicine from a description of single entities to a functional description of the whole system, thus revolutionizing our understanding of complex biological regulatory systems and providing major new opportunities for practical applications.

There are three essential and necessary steps in achieving this aim: (1) identifying the entities of the biological system, (2) describing the dynamic behavior of these entities with respect to time and under external stimuli, and (3) describing the interactions among these entities. Ultimately, this information can be combined into a model that is not only consistent with current knowledge but provides new insights and predictions, such as the behavior of the system in conditions that were previously unexplored.

Molecular biological networks, in particular gene networks are frequently in the focus of interest. A network may be defined as a set of interactions, generally represented by edges or arrows, between variables, which may be referred to as elements or entities, generally represented by nodes. Gene expression is finely regulated within the cell both at transcription and translation levels and this control is essential for maintaining cell homeostasis and to allow organisms to live. The causal relationships between the transcription of a set of genes is usually represented by a Gene Regulatory Network (GRN). A full description of a GRN implies the identification of the genes comprised in it, the comprehension of the gene connections (functional relations) and the elucidation of the kind of relationships between the genes of the GRN, i.e. facilitatory or inhibitory relationships. A GRN does not represent the details of the whole set of events that connect, for example, the transcription of two genes. However, the correct description of a GRN is of great importance since it allows either predicting the behavior of the system following perturbations, or manipulating it in the direction of a specific aim.

The modeling of a GRN based on experimental data is also called reverse engineering or network inference, and it is a challenging task as the problem itself is of a combinatorial nature, in particular, finding the right combination of regulators. Available data can be scarce and inaccurate. Various informatics approaches have been used or specifically proposed in order to identify gene networks.

Gene network inference algorithms

Algorithms proposed in the literature to solve the network inference problem from observational data can be classified in four main classes, according to the mathematical formalism they use:

- Co-expression networks and clustering algorithms, of which the output is generally an undirected graph.
- Bayesian networks, of which the output is generally a directed graph.

A Bayesian network is a Directed Acyclic Graph (DAG) whose nodes correspond to random variables. The directed edges between the nodes represent conditional dependency between the connected nodes, whereas the absence of an edge represents conditional independency. Each node has a probability function that associates the value of the parent nodes in the DAG with the value of the children nodes. Generally, the Bayesian Network represents the joint probability distribution on the variables. Once defined, the Bayesian Network can be used to compute the probability of a variable given a set of values for some of the others. Bayesian networks model probabilistic dependencies among variables and not causality, that is, the parents of nodes in the network are not necessarily also the direct causes of its behavior. In fact, conditional dependency is not equivalent to a causal relationship.

- Information-theoretic approaches, of which the output is generally a directed graph. Information-theoretic approaches use a generalization of pairwise correlation coefficients called Mutual Information, to compare expression profiles from a set of microarrays. Information-theoretic approaches can deal with steady-state gene expression data, or with time-series data as long as the sampling time is long enough to assume that each point is independent of the previous points. As in the case of Bayesian network, edges in the network do not represent a direct causal interaction between two genes, but only a statistical dependency.
- PC algorithm, of which the output is generally a directed graph.

There have been studies in the scientific literature reporting the use of the so-called PC algorithm on gene

expression data with the aim of reconstructing GRNs. The PC algorithm, developed by Peter Spirtes and Clark Glymour Spirtes (P., Glymour, C. and Scheines, R. (1993), Causation, Prediction and Search, Springer Verlag, Berlin, or Spirtes, P., Glymour, C., & Scheines, R. (1991), An algorithm for fast recovery of sparse causal graphs, Social Science Computer Review, 9, 62-72) is a causal relationship discovery algorithm. It is based on the theoretical framework of inductive causation, initially proposed in Verma and Pearl Verma (T.S. & Pearl, J. (1990), Equivalence and synthesis of causal models, Proceedings of the Conference on Uncertainty in Artificial Intelligence (pp. 220-227), Cambridge, MA) aiming to discover causal structures in purely observational data. Although initially proposed in the research domain of the social sciences, it has also been applied to the biological domain, in particular to the problem of GRN inference, due to its ability to find very sparse networks also when the number of variables is much larger than sample size.

Wimberly et al. (F.C. Wimberly, T. Heiman, J. Ramsey, C. Glymour, Experiments on the accuracy of algorithms for inferring the structure of genetic regulatory networks from microarray expression levels, Proc. IJCAI 2003 Bioinformatics Workshop) applied the PC algorithm on microarray measurements of a time series of yeast synchronized cells. The analysis was restricted to 11 genes that are reported in the website http://staffa.wi.mit.edu/cgi-bin/young_public/navframe.cgi?s=17&f=structures. In this trial the PC algorithm did not perform better than chance (error rate range: 0.47-0.52), as did the other machine learning algorithm tested therein.

Wang et al. (Wang M., Benedito V.A., Zhao P.X., Udvardi M. (2010), Inferring large-scale gene regulatory networks using a low-order constraint-based algorithm, Molecular BioSystems 6, 988-998) proposed a modified version of the original PC algorithm called Low-order PC-algorithm (LPC algorithm) which computes only low-order Conditional Independence (CI) tests rather than full-order CI tests. Although this modification improves substantially the computational efficiency, the authors find most of the causal relationships in their case studies. LPC algorithms are computationally scalable to high-dimensional datasets, do not require a discretization of transcript levels, and can predict causal relationships. The algorithm is an alternative to Bayesian network learning methods for large-scale GRN reconstruction.

Zhang et al. (Zhang XJ , Zhao XM, He K, Lu L, Cao YW, Liu JD, Hao JK, Liu ZP, Chen LN. (2012), Inferring gene regulatory networks from gene expression data by path consistency algorithm based on conditional mutual information, Bioinformatics 28, 98-104) used a modified version of the original PC algorithm called Path Consistency Algorithm with Conditional Mutual Information (PCA-CMI). The authors tried to combine the pros of the PC algorithm with testing of Conditional Mutual Information (MI), which in effect enables measurement of non-linear dependency. The original PC-algorithm measures the Pearson correlation coefficient between gene pairs instead. With the general hypothesis of Gaussian distribution underlying gene expression data, CMI between a pair of genes is computed by a concise formula involving the covariance matrices of the related gene expression profiles. In addition, in order to improve the robustness and the accuracy of the inference in large-scale datasets, the analysis is performed in a modular fashion.

Kalisch et al. (Kalisch M., Buhlmann P. (2007), Estimating High-Dimensional Directed Acyclic Graphs with the PC-Algorithm, Journal of Machine Learning Research 8, 613-636, and in M. Tan, M. Alshalalfa, R. Alhajj, and F. Polat. (2008), Combining Multiple Types of Biological Data in Constraint-Based Learning of Gene Regulatory Networks, Proc. IEEE Fifth Symp. Computational Intelligence in Bioinformatics and Computational Biology (CIBCB ’08), pp. 91-98) used a modified version of the original PC algoritm called PC algorithm for Partially-Dense graphs (PCPDPr). In this modified application of the PC algorithm the authors showed that combining more than one type of data in GRN modeling increases the quality of the derived network. The algorithm is a modified version of the original PC called PC algorithm for Partially-Dense graphs (PCPDPr). The PCPr algorithm of Kalisch et al. successfully combines two different kinds of data (the microarray and binding location data) through conditional independence tests. However, one drawback of PCPr is that the maximum order may increase to a large value, and this may lead to errors because of the low power of conditional independence tests for high orders.

Tan et al. (M. Tan, M. Alshalalfa, R. Alhajj, and F. Polat. (2008), Combining Multiple Types of Biological Data in Constraint-Based Learning of Gene Regulatory Networks, "Proc. IEEE Fifth Symp. Computational Intelligence in Bioinformatics and Computational Biology (CIBCB ’08), pp. 91-98) proposed a PCPDPr algorithm which makes use of prior knowledge from another type of data to identify dense nodes in GRNs. This method requires identifying dense nodes a priori and performs a greedy search procedure for finding conditioning sets, sometimes referred to as separators. Their PCPDPr outputs a better network than the other algorithms in a shorter time. The task of expanding an established molecular biological network, especially GRN expansion has received less attention than networks reconstruction. However, in the biological practice the situation of molecular biological network expansion is more frequent. In fact, a biologist working on a research topic has usually prior knowledge about relevant entities and possibly involved sub-networks and/or can formulate reasonable hypothesis about putative interesting networks.

US 2009/0063376 relates to gene regulation network construction using time series microarray data under the method of the Bayesian network. Prior knowledge is incorporated to help genetic algorithm search. For example, if it is known from biologists or from previous analyses that gene i regulates gene j, arcs i->j in the initial population of the genetic algorithm are hardcoded. In this way, as knowledge accumulates, the reconstructed networks may be expanded from sub-networks to networks, from networks for a particular biological process to networks for multiple biological processes, etc.

Hodges et al. (Andrew P. Hodges, Dongjuan Dai, Zuoshuang Xiang, Peter Woolf, Chuanwu Xi, Yongqun He (3.2010), Bayesian Network Expansion Identifies New ROS and Biofilm Regulators, PLoS ONE www.plosone.org (Volume 5, Issue 3, e9513)) reconstructed pathways and uncovered missing connections from experimental data using a compendium of microarray gene expression data obtained from *Escherichia coli,* and constructed a series of Bayesian network models for the reactive oxygen species (ROS) pathway as defined by EcoCyc. The authors propose an expansion algorithm to identify those genes that provide the best network score when added to an existing core network topology. The method described therein considers the expansion of at one node at a time and independently from the other nodes.
An object of the invention is, given an established causal molecular biological network, to provide a method for determining entities that are significantly involved with or related to entities in the given network.
Another object of the invention is to provide a method for determining suitable entities for expanding an established causal molecular biological network, especially for expanding an established gene network.
Another object of the invention is to provide a method for determining significant causal relationships between entities of an established causal molecular biological network and candidate entities, especially for an established gene network.

DISCLOSURE OF THE INVENTION

In a first aspect, the present invention provides a method for determining suitable entities for expanding an established causal molecular biological network, the method comprising:

A) Providing an established causal molecular biological network, a candidate set of entities and abundance data thereof;
B) Generating at least one intermediate expansion of said established causal molecular biological network with a defined part of the candidate set of entities by use of a computer program and by using the abundance data;
C) Determining a measure of interrelationship for every entity of the candidate set of entities with respect to at least some of the entities of the established causal molecular biological network;
D) Selecting entities out of the candidate set of entities based on the determined measure of interrelationship.

The above method provides an identification of those new entities that are significantly related to the known entities of the established causal molecular biological network. These new entities may be called expansion entities. They are preferably obtained by analyzing all entities of interest. The identified suitable entities from the candidate set of entities, sometimes referred to as a candidate list, thus may serve to provide knowledge to the behavior of an explored system with respect to time, under external stimuli and/or in the explored conditions. The explored system may either refer to the established causal molecular biological network itself or to the candidate set of entities or both together.
According to preferred embodiments, the candidate set of entities may contain partial or whole genomes or partial or whole metagenomes. The feature "providing the candidate set of entities" may thus be understood as providing defined portions of genomes or whole genomes or providing defined portions of metagenomes or whole metagenomes. In particular, if the molecular biological system of interest is a genome containing several thousands of genes (for example, 25,000 genes or 30,000 genes), then the feature "providing the candidate set of entities" may be interpreted as providing a portion, i. e. a tile of the genome comprising at least two, preferably a few hundred or a few thousand genes (for example, 1,000 genes). Thus, the method permits, by means of division in several subtasks (i.e. 25 or 30 subtasks in the examples above), to apply the computer program generating the intermediate expansions slice by slice to the whole genome. If the molecular biological system, as, for example, the genome in question, is of a smaller, adequate size, then the feature "providing the candidate set of entities" may also be interpreted as providing the whole genome as input for steps B), C) and D) of the method.
According to a preferred embodiment, the candidate set of entities is formed by the whole genome of an organism and the part of the candidate set of entities is formed by a slice of the genome with "s" entities. At least one, preferably several intermediate expansions are generated with those "s" entities, resulting in a statistically sensible or significant of both measure of interrelationship for every one out of the "s" entities of the candidate set of entities with respect to at least some of the entities of the established causal molecular biological network. The entities with the most prominent, or significant, or both, value of interrelationship are selected out from the "s" entities. Preferably, the measure of interrelationship is determined by calculating a quotient of a number of times that said entity has a direct or indirect binding with entities from the established causal molecular biological network and a number of appearances in the intermediate expansions. An indirect binding between two entities can be defined by an absence of direct binding and by a presence of chains connecting the entity with the entities from the established causal molecular biological network. In some embodiments, a chain connecting an entity indirectly with another entity from the established causal molecular biological network may be limited to a maximum number of entities, whereas in other embodiments an indirect binding may qualified as established if a single chain connecting the entity with the entities from the established causal molecular

biological network can be detected.

According to a preferred embodiment, said selection in step D) comprises comparing the measure of interrelationship for every entity with a threshold value derived from a measure of interrelationship of entities from the established causal molecular biological network. Preferably, entities will be selected, if their measure of interrelationship is above or equal to the threshold value. Alternatively, selecting entities according to step D) can be done by sorting the candidate set of entities according to a value of the determined measure of interrelationship and to select a defined number of entities, e. g. 1, 10, 100 or 1,000, out of the sorted candidate entity list. The defined number of entities can be displayed or printed out or presented to a user in any possible way. Thus, advantageously, a-priori information of the established causal molecular biological network can be used to select the entities. This a-priori information of the established causal molecular biological network is related to the relationships among the entities of the established causal molecular biological network.

According to another aspect, the invention provides a method for expanding an established causal molecular biological network, wherein entities from a candidate set of entities are selected according to a method as described above and wherein the selected entities are added to the established causal molecular biological network. Advantageously, the invention thus provides a method of network expansion. There can be a user's interaction for choosing some of the selected entities or it can be done automatically with all the selected entities. The number of entities added to the network may be arbitrary, e. g. 1, 2, 5, 10, 50, 100 or 1,000.

According to another aspect, the invention provides a method for determining significant causal relationships between entities of an established causal molecular biological network and candidate entities, the method comprising:

A) Providing an established causal molecular biological network, a candidate set of entities and abundance data thereof;
B) Generating at least one intermediate expansion of the said established causal molecular biological network with a defined part of the candidate set of entities by use of a computer program and by using said abundance data;
C) Determining a measure of interrelationship for every pair of entities, wherein one entity is from the candidate set of entities and a second entity is from said established causal molecular biological network;
D) Selecting pairs of entities based on the determined measure of interrelationship.

The candidate set of entities may contain partial or whole genomes or partial or whole metagenomes as described above. The invention thus provides a method for discovering relationships between the entities of the established causal molecular biological network and the entities of the candidate set of entities.

According to another aspect, the present invention provides a method for determining significant causal relationships between entities of an established causal molecular biological network, the method comprising:

A) Providing an established causal molecular biological network, a part of the established causal molecular biological network, a candidate set of entities and abundance data thereof;
B) Generating at least one intermediate expansion of the part of said established causal molecular biological network with a defined part of the candidate set of entities by use of a computer program and by using said abundance data;
C) Determining a measure of interrelationship for every pair of entities, wherein one entity is from the candidate set of entities and a second entity is from said part of said established causal molecular biological network;
D) Selecting pairs of entities based on the determined measure of interrelationship.

According to a preferred embodiment, a subset of the entities from the established causal molecular biological network forms the candidate set of entities and the remaining entities of the established causal molecular biological network form the part of the said established causal molecular biological network. The invention thus provides a method for discovering relationships within the established causal molecular biological network.

According to another preferred embodiment of the invention, a subset of the entities from the established causal molecular biological network forms just a fraction of the candidate set of entities and the candidate set of entities contains further entities whose relationships within the established causal molecular biological network are of interest. The remaining entities of the established causal molecular biological network form the part of the said established causal molecular biological network.

In these embodiments, preferably, the measure of interrelationship is determined by counting the number of times the entities from the pair of entities are connected to each other in the intermediate expansions.

According to a preferred embodiment, said selection in step D) comprises comparing the measure of interrelationship for every pair of entities with a threshold value derived from a measure of interrelationship of pairs of entities from the established causal molecular biological network or from the part of said established causal molecular biological network. Preferably, pairs of entities will be selected, if their measure of interrelationship is above or equal to the threshold value. In other words, selecting entities according to step D) can be done by sorting the pairs of entities according to a

value of the determined measure of interrelationship and to select a defined number of pairs, e. g. 1, 10, 100 or 1,000, out of the sorted list. The defined number of pairs can be displayed or printed out or presented to a user in any possible way. Thus, advantageously, a-priori information of the established causal molecular biological network can be used to select the pairs of entities. This a-priori information of the established causal molecular biological network is related to the relationships among the entities of the established causal molecular biological network.

In all methods as described, step B) comprises applying a causal network discovery algorithm to the entities. Most preferably, step B) comprises applying the PC algorithm. Using the PC Algorithm has the advantage that it focuses directly on the causal relation between the entities and not on the dependency as would do a Bayesian network learning algorithm. The PC algorithm does not require the definition of probability functions and computation of quantities which are necessary for checking the consistency of the networks with respect to the available data as would do a Bayesian networks learning algorithm. In the PC algorithm, causal relationships between entities are discovered by means of conditional independence tests. The PC-algorithm is designed to recover directed acyclic graphs using iterative tests of conditional dependence and independence, with the effect that it finds also joint regulations and not only pair-wise regulations. Therefore, the output of the algorithm is a network comprising causal relationships. The edges in the resulting graphs of the intermediate expansions do not comprise statistical values, say they are not afflicted by statistical values.

Since the PC algorithm starts from a complete graph and assuming the worst case, for which no edges are removed by independence tests, the running time of the algorithm is exponential over the number of the entities of the graph. PC algorithm is computationally feasible scaling to a few thousands of entities for sparse networks and has competitive performance with other directed acyclic graph reconstruction algorithms. Since the molecular biological networks in question show only a small number of edges, and since the algorithm is supposed to be applied to graphs that are sparse, this worst case is unlikely to occur. The running time of the algorithm has been found to be much smaller than exponential when applied to these molecular biological networks.

Preferably, the PC algorithm is used in the case when the molecular biological network is a gene regulatory network. The PC algorithm applied to expand an established GRN provides a advantageous approach for GRN reverse-engineering from gene expression data. The invention, thus, also provides a solution to the problem of complexity of a genome-wide application of PC, as the technical improvement solves the problems of computational cost of applicability of PC algorithm.

In all methods as described, preferably, step B) comprises dividing the candidate set of entities into a defined number $N_{SUB}$ of subsets each having a defined number "s" of entities and establishing $N_{SUB}$ intermediate expansions. Especially, if the candidate set of entities contains more than the number "s" of entities for the generation of the intermediate expansions, then the candidate set of entities is preferably divided into subsets which contain a number of entities which is equal to or less than the number "s" of entities for the generation of the intermediate expansions. The candidate list of entities is divided in $N_{SUB}$ subsets, which may also be called "tiles" from here on, containing each a number "s" of entities, which can also be referred to as a size of the tile or as a subset size. Preferably, the subset size "s" is greater than 100, greater than 1,000, preferably a defined number between 100 and 10,000, most preferably between 1,000 and 5,000.

Advantageously, the network discovery algorithm, in particular the PC algorithm, considers the causal relationship of a node in a broad contest of a part of variables, namely the defined part of candidate entities, that is the number "s" of entities for the generation of the intermediate expansions. Thus the network discovery algorithm will discover the causal relationship with a network-correlated single candidate entity when it detects a conditional independency between the single candidate genes, one or more of the other candidate genes and the entities of the established causal molecular biological network. The result for each entity is influenced by the whole set of the candidate entities.

In some embodiments, determination of the subset size "s" may also be addressed by running the intermediate network expansion procedure several times with different values of the parameter "s". The parameter may then be chosen by analyzing ROC curves and Precision-Recall curves charted with the results.

In embodiments, where Step B) comprises applying the PC algorithm, the number of entities in the tile may be generally limited to a number $s_{max}$ of entities, the PC algorithm being efficiency-related limited to this number of entities. The number $s_{max}$ may be a defined number between 100 and 10,000, most preferably between 1,000 and 5,000.

In all methods as described, preferably, step B) is iteratively repeated for a number $N_{iter}$ of iterations. The iterative approach increases the quality of the results. The iterative approach permits increasing the precision and to have as a parameter the number $N_{iter}$ of iterations, which can function as a threshold that controls the tradeoff between sensitivity and specificity. Because in the case of application of the causal networks discovery algorithm the statistics are not inherent to the output network, a controllable statistical parameter may be provided by the number of intermediate expansions, that is the number $N_{iter}$ of iterations. Therefore, in the context of the invention, the methods according to some preferred embodiments may also be called PC-Iterative Methods (PC-IM). The iterative term indicates that the analysis of the part of the candidate set of entities is repeated several times.

According to a preferred embodiment, the PC-Iterative Method (PC-IM) produces a list of genes that expands an established entity network by considering all the genes of the candidate set and evaluating if their gene expression

is likely to be in causal relationship with the expression level of one or more genes present in the established entity network. The evaluation is carried out by the iterative and repeated application of the PC-algorithm to subsets of the candidate list of genes.

In all methods as described, according to a preferred embodiment, a threshold value may be determined by applying the steps A), B) and C) to a subset of entities from the established causal molecular biological network forming the candidate set of entities. The remaining entities from the established causal molecular biological network form a part of said established causal molecular biological network. At least one intermediate expansion of the part of said established causal molecular biological network with a defined part of the candidate set of entities by use of a computer program and by using said abundance data is generated; and a measure of interrelationship for every entity of the candidate set of entities with respect to at least some of the entities of the established causal molecular biological network or a measure of interrelationship for every pair of entities, wherein one entity is from the candidate set of entities and a second entity is from said established causal molecular biological network or from the part of said established causal molecular biological network is determined. Iteratively repeating of step B) may increase the precision, as described above. The fact that an established network is known, thus, permits to draw performances curves for the determination of the threshold. The established causal molecular biological network is divided in numerous random sub-networks and these sub-networks are used for establishing a so-called gold standard of the established causal molecular biological network. Determination of the threshold value may also serve to intrinsically evaluate the PC-IM result. In this way, the method assesses its own performance and provides an estimate on the precision of the final expansion result. Advantageously, the threshold can be determined using the output data of the application of PC-IM at Step B) without any additional application of PC-IM or of any other causal network discovery algorithm used in Step B) of the methods as described above. The advantage over a more usual procedure that requires additional applications is crucial due to the computational burden of applying such algorithms.

According to an aspect of the invention a method for finding candidate targets for a drug or for determining an impact of a drug or for detecting conspicuous cells or for crop breeding is provided, wherein the steps of one of the methods as described above are carried out.

Applications of the methods, especially of expanding a known gene regulatory network (GRN), comprise extending, for a given drug, the number of druggable candidate targets. The candidate set of elements, the abundance data or the established causal molecular biological network may be related to observations of a molecular biological system under drug impact or may comprise prior knowledge of the molecular biological system under drug impact. Providing suitable entities from the candidate list and/or pairs of highly interrelated entities according to the methods as described above, may provide knowledge on the behavior of the respectively explored system under drug impact. This may also lead to a better understanding of the metabolic effects of a treatment/drug. The candidate set of elements, the abundance data or the established causal molecular biological network may also be related to observations of a disease. Providing suitable entities from the candidate list and/or pairs of highly interrelated entities according to the methods as described above, may provide knowledge on the explored disease.

Applications of the methods, especially of expanding a known GRN, comprise detection and monitoring of conspicuous cells. The candidate set of elements, the abundance data or the established causal molecular biological network may be related to observations of a possibly infected molecular biological system having conspicuous cells or may comprise prior knowledge of the possibly infected molecular biological system. If a disease is identifiable by a significant presence of one or several defined entities out of the candidate set of entities or by a presence of one or several highly interrelated pair of entities, the conspicuous cells may be detected and monitored over the time. Providing suitable entities from the candidate list and/or the pairs of highly interrelated entities according to the methods as described above, may thus serve to identify and observe infected biological systems. Furthermore, this may also serve to accumulate knowledge for understanding the impact of diseases in the explored system.

Applications of the methods, especially of expanding a known GRN, comprise crop breeding and genetically modifying organisms. The candidate set of elements, the abundance data or the established causal molecular biological network may be related to observations of a molecular biological system under heredity transmission of genes or may comprise prior knowledge of the molecular biological system. Providing suitable entities from the candidate list and/or the pairs of highly interrelated entities according to the methods as described above, may provide knowledge on the components of the explored system under breeding.

According to a another aspect of the invention, a computer program product, which is directly loadable into the internal memory of a digital computer, comprises software code portions suitable for implementing one of the methods as described before when said product is run on a computer. The computer program product may be a computer program preferably stored on a machine readable storage medium like RAM, ROM, or on a removable and/or portable storage medium, such as, but not limited to a CD-ROM, flash memory, DVD, BlueRay, FlashDisk, a storage card or a USB-stick. The computer program may be provided on a server to be downloaded via for example a data network such as the internet or another transfer system such as a phone line or a wireless transfer connection. Additionally, or alternatively, the computer program product may be a network of computer implemented computer programs such as on a client/server

system or a cloud computing system, an embedded system with a computer program or on an electronic device, like a smart phone or a personal computer on which computer programs are stored, loaded, run, exercised, or developed.

According to a another aspect of the invention an apparatus, especially suitable for implementing any one of the methods as described above, for determining suitable entities for expanding an established causal molecular biological network, for expanding an established causal molecular biological network and/or for determining causal relationships between entities comprises:

- an input means for providing an established causal molecular biological network, a candidate set of entities and abundance data thereof;
- a unit for generating intermediate expansions of said established causal molecular biological network or of parts of said established causal molecular biological network with defined parts of the candidate set of entities by using said entity abundance data; - a unit for determining a measure of interrelationship for entities or pairs of entities and
- a unit for selecting entities or pairs of entities based on the determined measure of interrelationship.

It is to be understood that the definitions and explanations of the methods, measurements and terms referred to in this description apply mutatis mutandis for all aspects described in this specification in the following except as otherwise indicated.

The following Figures and Examples are intended to illustrate various embodiments of the present invention. As such, the specific modifications discussed therein are not to be understood as limitations of the scope of the invention. It will be apparent to the person skilled in the art that various equivalents and modifications may be made without departing from the invention, and it is thus to be understood that such equivalent embodiments are to be included herein.

BRIEF DESCRIPTION OF THE DRAWINGS

Embodiments of the invention are shown in the drawings and will be described in the following.

Figure 1 depicts an apparatus according to the invention;
Figure 2 is a diagram showing principal steps of a network expansion method;
Figure 3 is a flowchart showing a subsets generation procedure;
Figure 4 is a flowchart showing an iterative subsets generation procedure;
Figure 5 is a flowchart showing an application procedure;
Figure 6 is a flowchart showing a network expansion analysis procedure;
Figure 7 is a flowchart showing a procedure to determine a threshold frequency and a final list of entities that expand a network;
Figure 8 is a flowchart showing an overall network expansion procedure;
Figure 9 is a flowchart showing an established entity network relationships analysis procedure;
Figure 10 is a flowchart showing a procedure for determination of a threshold frequency ratio and of a final list of pairs of entities in causal relationship with the network;
Figure 11 is a flowchart showing an overall causal relationships discovery procedure;
Figure 12 is a flowchart showing an internal-external relationships analysis procedure;
Figure 13 is a flowchart showing a procedure to determine a threshold frequency ratio and a final list of internal-external pairs of entities in causal relationship;
Figure 14 ian overall network expansion and internal-external causal relationships discovery procedure;
Figure 15 shows a ROC curve;
Figure 16 shows a PR curve;
Figure 17 shows performance curves;
Figure 18 shows a ROC curve;
Figure 19 shows a PR curve.

DETAILED DESCRIPTION OF THE EMBODIMENTS

Prior to discussing specifics of systems and methods for determining suitable entities for expanding established causal molecular biological networks and for determining significant causal relationships between entities of established causal molecular biological networks and candidate entities, it may be helpful to briefly define a few terms as used herein. The following definitions are not intended to be limiting, but may comprise alternate definitions commonly utilized by those of ordinary skill in the art. Additionally, the following list of definitions is not intended to be exhaustive, but rather discuss a few key terms that may be helpful to those of skill in the art.

A "molecular biological network" may comprise any graph, database, or other data file suitable for identifying

a plurality of molecular biological entities and relationships between entities. The molecular biological entities form the variables or entities of the graph. The graph may comprise a system-wide representation of some or all biological systems within a prokaryotic or eukaryotic organism, in particular within a virus, a fungi, a bacterium, a microorganism, a yeast, a cell or cell tissue, a plant, an animal body or the human body. The graph may comprise a plurality of molecular biological entities, such as genes, proteins, metabolites, hormones, enzymes, aminoacids, transporters, or other suitable entities, and each entity may be associated with one or more other entities via a relationship. In particular, the established causal molecular biological network can comprise a gene regulatory network, a protein network, a metabolite network, a transcript network or a hybrid network, which may be a composite network made of genes, proteins transcripts or metabolites. The entities of the established causal molecular biological network and of the candidate set of entities can be genes, proteins, enzymes, hormones, amino acids, transcripts or metabolites, or mixtures thereof, accordingly. The established causal molecular biological network is composed of nodes representing the genes, proteins, enzymes, hormones, amino acids, transcripts and/or metabolites and edges representing molecular interactions such as protein-DNA, protein-RNA, DNA-DNA, RNA-RNA, and protein-protein interactions or rather indirect relationships between the entities

The term "abundance data" as used herein generally refers to any information relating entities of molecular biological networks with each other. It may in particular refer to concentration data or to relative concentration data of the entities. It may in particular refer to "expression data". The term "expression data" refers to any information related to the chain of events that encompasses transcription, splicing, post-transcriptional regulation, translation or post-translational modification. A variety of phenomena can be represented in a simplified way as a causal relationship between the transcription levels of genes. Essential regulators of the transcription step are DNA binding proteins called transcription factors which act on the promoter region of genes and thereby modulate their transcription. The promoter region of a gene generally refers to any kind of regulatory DNA sequence that is operably linked to a downstream coding sequence, wherein said promoter is capable of binding RNA polymerase and initiating transcription of the encoded open reading frame, thereby driving the expression of said downstream coding sequence. Transcription of a gene or of a gene cluster encompassing several genes, or of several individual genes is a highly regulated event within the cell or within the organism that can be stimulated and/or inhibited by a variety of factors. In particular, the expression of genes, and thereby the generation of gene expression data, can be analyzed and measured with all the available techniques know in the art, including, but not limited to, micro-array analysis, analysis of protein expression, analysis of mRNA expression and translation, or by RNA sequencing. Methods for analyzing gene expression are routine methods in the art known by the skilled person. Expression data can be measured on whichever subcellular fraction, especially on total mRNA, on total protein levels, or only on polysomal fractions.

Due to the fact that transcript profiling can be performed on a genome-wide scale by use of different techniques such as microarrays, serial analysis of gene expression (SAGE), massively parallel signature sequencing (MPSS) and, more recently, by direct sequencing of transcripts by high-throughput sequencing technologies (RNA-Seq), inference of gene regulatory networks (GRN inference) has been often carried out upon starting from gene expression data. The availability of large amounts of transcriptional data generated by microarray experiments and, more recently, by so-called Next Generation Sequencing (NGS), has so far posed the task of reverse engineering the GRNs and the gene connecting pathways by inference from these types of data. Unlike gene regulatory networks analysis, proteome and/or metabolome network analysis is still challenging and far from being a routine approach.

"Gene expression data", in particular, can be originated by the suppression or on the enhancement of the expression of one or more genes using transgenesis, natural mutants or a treatment, e.g. a drug, and the measurement of how the other gene's expression is influenced, e. g. by experimental perturbed data. Within this type of data, firstly, one may consider time series data in the specific case of frequent sampling, namely when the following time point is dependent from the one before. The method has proved fruitful in unraveling small parts of a regulatory network. Time series microarray data, however, has the disadvantage that it is scarce and in practice not sampled at high frequencies, such that causality may be lost from one time point to the other. Secondly, one may also consider data from transgenic experiments. A drawback of this kind of data is that it may provide information only about the effects of one or few manipulated genes. Moreover, in complex organisms, as humans and plants, experimental data are difficult to obtain due to the long time to collect it and due to ethical issues. In preferred embodiments of the invention, "gene expression data" rely on the natural variation of expression levels of the same gene in different cells or other observational data. Advantageously, this may overcome the problems mentioned before for the experimental data. In this case the regulatory structure is inferred from the statistical dependencies and independencies among the measured expression levels.

The term "metagenome" as used herein, generally means any genetic material that is directly recovered from an environmental sample. The metagenome of the present invention may also be referred to as an "environmental genome", an "ecogenome" or a "community genome". One characteristic of the metagenome according to the present invention is its molecular biodiversity since it is not derived from the sequencing of a cultivated clonal culture such as, for example, a cultivated microorganism, but instead from the early environmental gene sequencing of a particular gene (often the 16S rRNA gene) from one or more natural sample(s), which allows for the establishment of a diversity profile in a natural sample.

The term "drug" generally means any composition formulated in solid, liquid (or gaseous) form which can be administered to a subject. Said composition generally comprises a therapeutically active compound optionally together with suitable auxiliary compounds such as diluents or carriers or further ingredients. In this context, it is distinguished between auxiliary compounds, i.e. compounds which do not contribute to the effects elicited by the compound upon application of the composition for its desired purpose, and further ingredients, i.e. compounds which contribute a further effect or modulate the effect of the therapeutic effective ingredient. Suitable diluents and/or carriers depend on the purpose for which the composition is to be used and the other ingredients. The person skilled in the art can determine such suitable diluents and/or carriers without further ado. Examples of suitable carriers and/or diluents include, but are not limited to, water, organic solvents, any kind of buffer, and salts.

The term "drug target" or "druggable target" generally refers to any kind of molecule which is responsive to a particular drug, i.e. any molecule which is either directly or indirectly influenced or affected by a drug. That is, a drug target may either directly bind a drug, or may be rather affected indirectly via protein-protein interactions, protein-RNA interactions, signal transduction pathways etc.

The term "conspicuous cells" generally means any cell which is abnormal, or does not comply with the characteristics of a standard cell.

The term "crop breeding" as used herein generally refers to the heredity transmission of genes by means of classical breeding methods that employ the crossing of closely or distantly related individuals to produce new crop varieties or lines with desirable properties. In particular, plants are crossbred to introduce traits or genes from one variety or line into a new genetic background.

The term "PC algorithm" may generally refer to an algorithm comprising a step with a conditional independence (CI) test for discovering causal relationships between the entities, and other steps for orienting the relationships between the entities. "PC algorithm" may in particular refer to the known algorithm as developed by Peter Spirtes and Clark Glymour Spirtes (P., Glymour, C. and Scheines, R. (1993), Causation, Prediction and Search, Springer Verlag, Berlin, or Spirtes, P., Glymour, C., & Scheines, R. (1991), An algorithm for fast recovery of sparse causal graphs, Social Science Computer Review, 9, 62-72).

Further definitions which are helpful to understand an exemplary embodiment of the "PC algorithm" as given below are the following:

Directed Acyclic Graph (DAG)

A graph G is an ordered pair <V,E> where V is the set of nodes and E is the set of edges linking the nodes. A directed acyclic graph (DAG) is a graph in which the edges have an orientation, usually indicated by arrowheads and in which there are no cyclic paths. A directed path from A to B in a graph G=<V,E> is a sequence of vertices beginning with A and ending with B such that for every pair of vertices <X,Y> consecutive in the sequence, the oriented edge (X Y)□ E. A path is cyclic when it contains a vertex more than once.

Conditional independence

Two random variables X and Y are said to be conditionally independent given a set Z of variables if $P(X|Y, Z) = P(X|Z)$ and $P(X|Z) \neq 0$, $P(YZ) \neq 0$, where $P(X|Z)$ denotes the conditional probability of X given Z; or, said in another way, when $P(X, Y|Z) = P(X|Z)\,P(Y|Z)$. This means that if X, Y are independent conditioned on Z then X does not provide any information about Y once given knowledge of Z and vice-versa.

Causal Markov condition

The causal Markov condition formalizes the intuition connecting causal graphs with the probability distribution they generate. Informally speaking, the Markov condition says that a variable in a DAG G=<V, E>, where each vertex in V represents a variable, is independent of its non-descendants given its parents. Let Pa (X) denote the set of variables of G having an arrowhead edge directed into X, and let it be called the set of parents of X. A descendant of X is any vertex Y such that there is a directed path from X to Y. Let De(X) denote the set of descendants of X. Let P be a probability distribution over the vertices in V generated by the causal structure represented by G. G and P satisfy the causal Markov condition if and only if for every W in V, W is independent of V \ (De (W )□Pa(W )) given Pa(W).

If P and G satisfy the causal Markov condition then we can decompose the joint probability distribution P (V) for the set of variables V as:

$$P(V)=\prod_{V\in V} P(V|Pa(V))$$

The causal Markov condition is the assumption that allows linking the causality and probabilistic interpretation of a DAG. In a causal DAG the causal Markov assumption states that given the values of a variable's immediate causes, the variable is independent of its earlier causes.

Causally sufficient

A set V of variables is said to be causally sufficient for a population if and only if in the population every common cause of any two or more variables in V is in V, or has the same value for all units in the population.

Faithfulness condition

A probability distribution P entailed by a causal graph G satisfies the faithfulness condition if and only if every conditional independence relation true in P is entailed by the causal Markov condition applied to G.

d-separation criterion

The Markov and faithfulness conditions imply a graphical characterization of conditional independence called d-separation (wherein the "d" stands for dependence).

This criterion is defined as follows:

Two variables X,Y are said to be d-separated by a node set Z if and only if every path between X and Y is blocked. A path is said to be blocked when there is an intermediate variable Z □Z such that: (1) the connection through Z is tail-to-tail or head-to-tail and Z has received evidence, or (2) the connection through Z is head-to-head and neither Z or any of Z 's descendants have received evidence.

Head-to-head connections also referred to as v-structures. Tail-to-tail, head-to-tail and head-to-head connections are represented in the following figure:

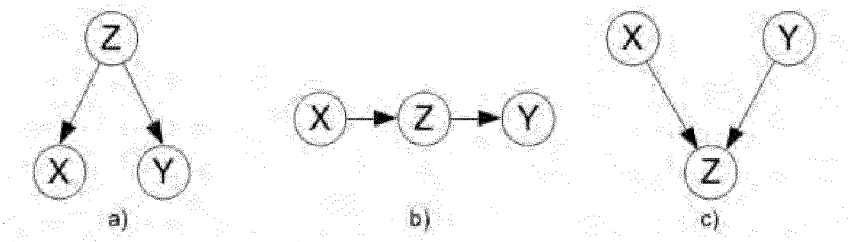


*Figure 2.3: in these simple graph each node represents a variable and arrowheads are oriented edges. From left to right we have: a) tail-to-tail connection $X \leftarrow Z \rightarrow Y$ ; b) head-to-tail connection $X \rightarrow Z \rightarrow Y$ and c) head-to-head connection $X \rightarrow Z \leftarrow Y$.*

Description of the PC algorithm

Assuming the causal Markov condition and faithfulness and causal sufficiency of a graph with respect to some given data, the PC-Algorithm recovers the causal structure of the variables described by the data. The PC-Algorithm works by progressively pruning the edges from a complete undirected graph built on the variables given in the data, until no more edges can be deleted, according to a function that decides when to delete the edge. The pruned graph, called the skeleton of the graph, is then oriented according to the d-separation rules.

A more detailed description of the algorithm is given with the help of the pseudo-code reported below.

```
PC Algorithm
Phase 1: find the skeleton by deleting edges between independent variables
1: Form a complete undirected graph G' from the set of nodes V
2: n = 0
3: repeat
4:   for all X ∈ V do
5:     for all Y ∈ Adj(X) do
6:       repeat
7:         choose an S ⊆ Adj(X)\Y and | S |= n
8:         if I(X, Y | S) then
9:           delete X − Y edge from G'
10:          save S in SepSet(X, Y) and SepSet(Y, X)
11:        end if
12:      until X − Y edge is deleted or all S have been chosen
13:    end for
14:  end for
15:  n = n + 1
16: until | Adj(X)\Y |< n
Phase 2: orient all possible v-structures
17: for all triple X − Z − Y where X ∉ Adj(Y) do
18:   if Z ∉ SepSet(X, Y) then
19:     orient triple as X → Z ← Y
20:   end if
21: end for
Phase 3: orient remaining unoriented edges using rules
22: repeat
23:   for all X → Y − Z where X ∉ Adj(Z) do
24:     orient X → Y → Z
25:   end for
26:   for all X − Y such that there is a directed path from X to Y do
27:     orient X → Y
28:   end for
29: until no more edges can be oriented
```

*Figure 2.4: pseudocode of the PC-Algorithm.*

As input the PC-Algorithm receives a set V of random variables. It works in three phases. The first phase starts by generating a complete undirected graph G from the set V, where each node of G represents a variable of V (line 1). From this point the variables are called nodes. Then the PC-Algorithm begins the pruning of the edges in the graph (lines 2 to 16). The following steps may be repeated, and at each repetition the value of the variable n is increased by one (line 15): for each node X in V, its adjacent nodes Adj(X) are collected and for each pair <X,Y>, with Y □ Adj ( X ) of adjacent nodes the statistical conditional independence between the nodes is checked (line 8). The tests for independence are conditioned on a set S of nodes. The nodes in S are selected only among the nodes that are adjacent to X, excluding Y (line 7). The size of the conditioning set S corresponds to the value of n, thus each time lines 3 to 16 are executed the algorithm carries out independence tests with a larger conditioning set. If the cardinality of the set of nodes adjacent to X is less than n, then the loop terminates. In other words, the PC-algorithm first sets the size of the conditioning set and then carries out the conditional independence tests for all and only the pairs of adjacent nodes. When independence between nodes X and Y given S is found, the edge connecting them is deleted and S is stored in the separation set SepSet (X,Y) of the pair <X,Y> (lines 8- 10). If all the pairs have been tested for independence, given all possible

separating set of size n (or at least all the separating sets used for conditioning), and if there are still some nodes adjacent in G, then the size n of the separating set is increased by one (line 15) and lines 4 to 14 are executed again, thus testing for independence the remaining adjacent pairs given a separating set larger by one with respect to the previous iteration. The algorithm stops iterating when the cardinality of the set of the nodes adjacent to X minus Y is less than n (line 16). At this point of the running, the skeleton of the undirected graph G is built, representing all the dependences between variables by edges between nodes. The separation sets SepSet(X,Y) for each pair of independent variables are stored.

Then the algorithm begins the orientation step, which can be divided into two phases. The former phase (lines 17-21), that is phase 2 of the overall algorithm, accomplishes the orientation of the v-structures of G. For all the triples of nodes <X,Y,Z> where X is adjacent to Z, Y is adjacent to Z, but X and Y are not adjacent (line 17), according to the d-separation criterion both edges between X and Z and between Y and Z can be oriented towards Z, only if Z is not in any separating set of the pair <X,Y> (lines 18-19).

Once all possible v-structures have been oriented, the algorithm enters its last phase (lines 23-29), namely phase 3, in which it orients all the undirected edges that were not oriented in v-structures. There are two cases that can occur and which can be resolved in order to respect the d-separation criterion: (1) for all the triples <X,Y,Z>, where there's an arrowhead from X into Y and an undirected edge between Y and Z, and where there is no edge between X and Z, the edge between Y and Z can be oriented towards Z (lines 23- 25); (2) for all the un-oriented edges between pairs <X,Y> , if there is a directed path from X into Y, the edge is oriented towards Y (lines 26-28). These two last orienting rules are repeated until no more edges in G can be oriented.

Figure 1 shows an apparatus 100 for determining suitable entities for expanding an established causal molecular biological network, for expanding an established causal molecular biological network and/or for determining significant causal relationships between entities according to the invention. The apparatus 100 comprises:

- an input means 110 for providing an established causal molecular biological network, a candidate set of entities and abundance data thereof;
- a unit 120 for generating intermediate expansions of said established causal molecular biological network or of parts of said established causal molecular biological network with defined parts of the candidate set of entities by using said entity abundance data;
- a unit 130 for determining a measure of interrelationship for entities or pairs of entities and
- a unit 140 for selecting entities or pairs of entities based on the determined measure of interrelationship;
- an output means 150 for presenting the selected entities or pairs of entities to a user.

The apparatus 100 may be of any type and form of computing device, such as a computer, network device or appliance capable of communicating on any type and form of network and performing the operations as described herein. The apparatus 100 may execute, operate or otherwise provide applications, which can be any type and/or form of software, program, or executable instructions. The apparatus 100 may comprise a computing device of any type, such as a desktop computer, portable computer, smart phone, tablet computer, or any other type of computing device. Computing devices in particular may be provided with a central processing unit, a main memory unit, an installation device, a network interface, display devices and input devices. The computing device may further comprise a storage device, such as one or more hard disk drives, for storing an operating system, application software programs, databases and software routines for executing the methods as described herein.

The apparatus 100 may include databases with the established causal molecular biological network and/or abundance information. In some embodiments, the databases may be stored on the apparatus 100, while in other embodiments, the databases may be stored on a data storage server, external storage device, within a cloud storage system, or otherwise accessible to the apparatus 100.

The input means 110 may allow a user in particular to input data, such as information on the established causal molecular biological network, a candidate set of entities and abundance data thereof. The apparatus 100 may also provide an interface allowing the user to provide queries, make selections or identifications of databases, entities or pairs of entities. Suitable input devices include keyboards, mice, trackpads, trackballs, voice interfaces, drawing panels and touchscreens. In some embodiments, the input means 110 may also communicate with servers for querying networks and/or abundance data. The apparatus 100 may comprise an application for accessing a server. In some embodiments, the application may comprise a web browser, while in other embodiments the application may comprise a dedicated application for communicating with servers.

In some embodiments, the apparatus 100 may generate one or more lists of entities. Such lists may be ordered, for example, by number such as a percentage values, or by identifiers of the entities, e. g. alphabetically. The apparatus 100 may provide lists, data tables, graphics, graphs, diagrams, charts or other output views for presentation via the output means 150 to the user. Output devices include in particular displays, speakers and printers. In one implementation, the information on the suitable entities for expanding an established causal molecular biological network, the expanded established causal molecular biological network and/or the determined causal relationships between the entities may

be provided on an output medium, stored in a memory means or transferred to a remote station. In one embodiment, the information is displayed on a screen or display as output medium.

In some embodiments the apparatus 100 may be part of a network environment comprising one or more local machines, which can be referred to as clients in communication with one or more remote machines, which can be referred to as servers via one or more networks, such as LAN, an Intranet, MAN, WAN, the Internet or the World Wide Web. The apparatus may include network interfaces to interface to the network through a variety of connections including, standard telephone lines, LAN or WAN links, broadband connections, wireless connections, or a combination of these. Connections can be established using a variety of communication protocols, such as Transmission Control Protocol (TCP/IP), Transport Layer Security (TLS) or any other network protocol.

With regard to figure 2, a network expansion method is described. The PC-Iterative Method (PC-IM) produces a list of genes that expands a known LGN by considering all the genes of an input list (usually the genes of a whole genome) and evaluating if their gene expression is likely to be in causal relationship with the expression level of one or more genes present in the LGN. The evaluation is carried out by the iterative and repeated application of the PC-algorithm to subsets of the input list of genes. Since the PC algorithm has efficiency-related limitation to the number of variables (1000 genes maximum) [Wang et al., 2010], the genes of the genome are divided in subsets (called tiles in the following) containing a number of genes (size of the tile) not greater than 1000. Moreover, the PC-IM has an intrinsic system to assess its own performance and this implies that it outputs also an estimate of the precision of the final expansion gene list.

Input data:

genes and relationships of the LGN that will be expanded;
list of genes of the genome;
expression data.

Parameters:

t: size of the tiles (number of genes in each tile);
i: number of iterations. This number specifics the times the whole genome is divided into tiles;
D: number of the subnetworks of LGN (subLGN);
d: size of the subLGNs.

The way PC-IM works is illustrated in Figure 2 and the whole procedure can be divided in five principal steps:

Step 1: Tiles creation

All the genes of the genome are randomly divided in non overlapping tiles of size t. Each tile is merged with the set of genes of the input LGN that PC-IM will expand. This operation is repeated times.

Adding the LGN genes to each tile permits to potentially infer the causal relationships of these genes with the other genes of the genome.

Step 2: PC application

PC runs on each tile using the gene expression data. As a result it gives a network (nodes and relationships) for each run. From these networks are extracted the subnetworks that include both the genes and relationships within the LGN genes and between LGN genes and the external ones, namely genes in the original tile and thus not included in the LGN.

Step 3: Frequencies computation

PC_IM creates a unique list of genes (called expansion list). This list contains all the genes present in at least one single subnetwork. For each gene in the expansion list, the algorithm calculates the frequency. This absolute frequency of a gene is the number of times that the gene is present in the subnetworks. The PC-IM computes both the absolute and normalized frequency. The latter is obtained dividing the absolute frequency of a gene with the number of times that the same gene can be found, namely it was present in a tile. Usually this number coincides with the number of iterations.

For example if i=100 and x gene has 90 of absolute frequency, then the normalized frequency will be 0.9.

Step 4: Intrinsic performance assessment

In this step PC-IM assesses its own performance and establish the minimum normalized frequency value necessary to have the best expansion performance. The assessment is done using the information of the original LGN.

Initially from the LGN are extracted D subLGNs of size d. With this operation the genes of the expansion list are split in three different categories:

subLGN genes
INTRA genes: genes of the LGN, that are not included in the subLGN
EXTRA genes: the additional genes randomly selected from the genome

The evaluation measures are calculated considering the expansion of each subLGN. A gene in the list will be considered to be positive if it is among the other genes of the LGN (INTRA) and negative if it is among the other genes (EXTRA). This operation is repeated for each of the D subLGNs and for each relevant frequency value. In practice, we use as Gold Standard the information on the LGN given in the input data. The evaluation measures are Positive Predictive Value (PPV or Precision), Sensitivity (Se) and False Positive Rate (FPR). Mathematically, they are defined by:

$$PPV = TPintra/(TPintra+FPextra) \quad (1)$$

$$Se = TPintra/(TPintra+FNintra) \quad (2)$$

$$FPR = FPextra/(FPextra+(TNintra+TNextra)) \quad (3)$$

where TP, FP, FN and TN are the number of the True Positive, False Positive, False Negative and True Negative respectively. The terms intra and extra have the same meaning specified above. Se and FPR corresponding to different frequencies are used to plot the receiver operating characteristic (ROC) curve and consequently the area under ROC curve (AUC). PPV and Se are used to plot the Precision-Recall (PR) curve and the minimum distance (dmin) from point (1,1) is calculated. This step returns the frequency that gives the maximum value of AUC and the minimum value of dmin..

Step 5: Cut-off frequency application.

In this last step PC-IM determines which genes, in the gene expansion list, will be returned as the final output of the method. The selection is carried out by using the frequency computed at Step 4 as a cut-off frequency on the gene expansion list computed at Step 3. The final list of genes is returned. The cut-off frequency is the minimum frequency that the genes, in the gene expansion list, must have in order to be considered as really involved in the expansion of the LGN.

In the following the main procedure of network expansion and of inference of relationships is presented and described in detail in flow charts depicted in figures 3 - 14. Although in the figures, a special case involving gene expression data is described, the data can be any kind of abundance data of elements from any molecular biological network. In the same way, the local gene network as mentioned in the figures 3 - 14 may be any molecular biological network that does not include all the entities.

In figure 3 an entity subsets generation procedure 300 is depicted. The entity subsets generation procedure divides the entities of an initial set of entities in subsets. In step 301, the initial set of entities of cardinality n is provided as an input data. In step 302, an established entity network with $n_{lgn}$ entities is provided as an input data. In step 303, from the initial set of entities the entities of the established gene network are subtracted. The remaining set has now $n-n_{lgn}$ entities. In step 304, a subset size "s" is provided as an input. In step 305, a remainder R of $(n-n_{lgn})/s$ is calculated, that is $R = (n-n_{lgn}) \bmod s$. In step 306 is tested if R = 0. If not, then in step 307 R entities are subtracted from the set and assigned to an additional subset. The set has now $n-n_{lgn}-R$ entities. In step 308, s-R entities are randomly selected and added to the additional subset without subtracting them from the set. In step 309, the additional subset of s entities are outputted. In step 310, a number N is calculated as $N = (n-n_{lgn}-R)/s$. If in step 306 the test is positive, then steps 307 to 309 are left out and the number N is calculated in step 310 immediately. In step 311, the entities of the set of $n-n_{lgn}-R$ entities are randomly partitioned into N subsets. In step 312, the N subsets of s entities are outputted. In step 313 is tested if R = 0. If not, then the number of subsets $N_{sub}$ set equal to N plus 1. If yes, then the number of subsets $N_{sub}$ is set equal to N. In step 316, the number of subsets $N_{sub}$ is outputted.

Thus, if the subset size "s" is a divisor of the number of entities in the initial set once subtracted the entities of the established entity networks, the subsets will not overlap each other and they are generated as a random partition of the list of $n-n_{lgn}$ entities. In the other case, there is a remainder R and an additional subset that partially overlaps with the others is generated from the established entity network. Each entity of the list of entities that is not an entity of the established entity network is present at least once and no more than twice in the system of $N_{sub}$ subsets that the procedure generates. The number of the subsets $N_{sub}$ is computed taking into account the value of the remainder.

In figure 4, an iterative entity subsets generation procedure 400 is depicted. In step 401, a number of iterations $N_{iter}$ is provided as input data. In step 402, a set of entities, an established entity network and a subset size "s" is provided as input data. In step 403, the entity subsets generation procedure 300, as described with regard to figure 3, is called, providing $N_{sub}$ random subsets of the entities present in the initial list of entities. In step 404, $N_{iter}$ is decremented by one. In step 405 is tested if $N_{iter}$ is equal to 0. If not, then steps 403 to 405 are repeated. If yes, then in step 406, a list of $M=N_{iter}*N_{sub}$ subsets each containing "s" entities are outputted. The number of iterations $N_{iter}$ thus specifies how many times the call is repeated. The iterative entity subsets procedure produces M subsets where M is equal to $N_{sub}$ times the number of iterations $N_{iter}$.

In figure 5, an application procedure 500 is depicted. In step 501, a set of entities, an established entity network, a subset size "s" and a number of iterations $N_{iter}$ are provided as input data. In step 502, the iterative entity subsets generation procedure 400, as described with regard to figure 4, is called, providing a list of subsets of the initial set of entities excluding the entities of the established entity network. In step 503, a number M is assigned to the dimension of the list of subsets. In step 504, the entities of the established entity network are added to the subset whose position in the list of subsets is M. In step 505, entity abundance data, comprising for instance gene expression data, is provided as input data. In step 506, a causal network discovery algorithm is applied to the entity subset whose position in the list of subsets is M with the correspondent expression data. In step 507, the number M is decremented by one. In step 508 is tested if M is equal to 0. If not, then steps 504 to 508 are repeated. If yes, then in step 509 M entity networks are outputted.

The entities of the established entity network are thus added to each subset and the resulting set of entities is fed into the entity network discovery algorithm that outputs the entity network comprising both the entities of the established entity network and the entities of the subset and putative causal relationship between their abundance levels. The application procedure outputs M entity networks. In figure 6, a network expansion analysis procedure 600 is depicted. In Step 601, M entity networks and an established entity network are provided as input data. In step 602, a list of all the entities in the M entity networks is computed. In step 603, from the list of entities in the M networks, the list of the entities of the established entity network is subtracted. In step 604, a number G is assigned to the dimension of the list of entities. In step 605, a number $F_G$ of times the entity in position G appears in the M entity networks is computed. In step 606, a number $f_G$ of times the entity in position G appears in the M entity networks and is also connected to an entity in the established entity network is computed. In step 607, a frequency ratio $f_G/F_G$ is computed. In step 608, the number G is decremented by 1. In step 609 is tested if G is equal to zero. If not, then steps 605 to 609 are repeated. If yes, then in step 610 the entity list with respect to the frequency ratio $f_G/F_G$ is ordered. In step 611, the ordered list of entities is outputted with the corresponding frequency ratios.

Thus, the network expansion analysis procedure 600 takes as input M entity networks and an established entity network. Initially, it builds a list with all the entities in the M entity networks that are not present in the established entity network. For each entity of the list it counts the number of times it appears in an entity network and the number of times it appears as connected to the established entity network. The ratio between the two numbers is called the frequency ratio $f_G/F_G$. The list of entities is ordered with respect to the frequency ratio and outputted.

A specific note may be given about the value of the parameter subset size "s". The parameter may be addressed by running the network expansion analysis procedure 600 several times with different values of the parameter "s". The parameter "s" may then be chosen over the ROC curves and Precision-Recall curves charted with the results as done for the value of the threshold frequency.

In figure 7, a procedure for determination of a threshold frequency ratio and of the final list of entities that expand the established entity network 700 is depicted. In step 701, M entity networks and the established entity network are provided as input data. In step 702, a number of replicas $N_{rep}$, and a dimension d of subsets of the established entity network are provided as input data. The dimension d must be less than the number of entities $n_{lgn}$ of entities in the established entity network. In step 703, a set D of d entities from the established entity network is randomly selected. In step 704, a sub-network of the established entity network is built with the entities in the set D and the edges that are also in the established entity network. In step 705, the network expansion analysis procedure 600, as described with regard to figure 6, is called with the established entity sub-network providing a list of entities with ordered frequency ratios. In step 706, the number of replicas $N_{rep}$ is decremented by 1. In step 707 is tested if $N_{rep}$ is equal to zero. If not, then steps 703-707 are repeated. If yes, in step 708, ROC curves are computed varying the threshold frequency ratio. In step 709, Precision-Recall curves are computed varying the threshold frequency ratio. In step 710, the curves are outputted. In step 711, a threshold ratio is provided as input data. In step 712, the network expansion analysis procedure 600, as described with regard to figure 6, is called with the established entity network providing a list of entities ordered

by frequency ratios. In step 713, the list of entities whose frequency ratio is above the threshold is computed. In step 714, the list of entities whose frequency ratio is above the threshold is outputted.

The procedure for the determination of the threshold frequency ratio and of the final list of entities that expands the network 700, thus, takes as input the M entity networks produced by the application procedure 500, as described with regard to figure 5. Initially, the procedure has the goal to determine a value of the threshold of the frequency ratio. This is done using the information of the established entity network. A sub-network of the established entity network is built from a random subset of the entities. The network expansion analysis procedure 600, as described with regard to figure 6, is then called passing the sub-network as parameter. The generation of the sub-network and the analysis of the M entity networks is repeated for $N_{rep}$ times. The entities in the established entity network that are not included in the sub-network are taken as positive entities and the entities that are not part of the established entity network are taken as negative entities. The number of true negatives, true positives, false negatives and false positives are computed and used to built ROC curves and Precision-Recall curves with varying values of the threshold frequency ratio. No additional application of the causal network discovery algorithm for building ROC and Precision-Recall curves is needed, because neither procedure 700 nor procedure 600 call the application procedure 500. By inspection of the curves, the user may choose the value of the threshold frequency ratio, or this can be done automatically. Finally, the network expansion analysis procedure 600, as described with regard to figure 6, is called with the established entity network as a parameter and the list of entities that are above the threshold frequency ratio are outputted.

In figure 8, an overall network expansion procedure 800 is depicted. In step 801, a set of entities, an established entity network, a subset size "s", a number of iterations $N_{iter}$ and expression data are provided as input data. In step 802, the application procedure 500, as described with regard to figure 5, is called. In step 803, a number of replicas $N_{rep}$ and a dimension d of the subsets of the established entity network are provided as input data. In step 804, the threshold frequency ratio is determined and the final list of entities that expand the network is determined according to procedure 700, as described with regard to figure 7. In step 805, the list of entities whose ratio is above the threshold is outputted.

The overall network expansion procedure 800, thus, takes as input a list of entities, an established entity network, the subset size, the number of iterations and the entity abundance data. The established entity network is a network that is supposed to be known and that the user would like to expand with some of the entities contained in the list of entities. The list of entities can include a whole genomic gene-set or just a part of it. The subset size is an integer number that represents the size of the subsets the list of entities will be divided into. The number of iterations is the number of times the division into subsets will be repeated. The abundance data is a table that contains one or several abundance levels for some or all the entities of the list and for some or all the entities of the established entity network. As a special case abundance data is expression data and abundance levels are expression levels. The overall networks expansion procedure 800 comprises the application procedure 500 and the procedure 700 that determines the threshold value of the frequency ratio and the final list of entities that expand the network. The overall network expansion procedure 800 outputs a list of entities whose abundance is likely to be in causal relationship with the abundance level of one or more entities present in the established entity networks. The user can take the list of entities whose ratio is above the threshold and add them to the established entity network or may choose some of the outputted entities to add or may make use of the knowledge as such.

In figure 9, an established entity network relationships analysis procedure 900 is depicted. In step 901, M entity networks and an established entity network are provided as input data. In step 901, furthermore, is specified, if the analysis of the relationships is bidirectional or not. In step 902, the list of all the entities in the established entity network is computed. In step 903, numbers N1 and N2 are set equal to the number of entities in the established entity network. In step 904, an empty list of pairs of entities is initialized. In step 905 is tested if the analysis of the relationships is bidirectional or not. If not, then in step 906 a number $F_r(N1, N2)$ of times the entity in position N1 is connected to the entity in position N2 in the M entity networks is computed. In step 907 a ratio $r(N1, N2) = F_r(N1, N2)/M$ is calculated. In step 908, the pair (N1, N2) with the ratio r(N1, N2) is inserted in the list of pairs. In step 909, the number N1 is decremented by 1. If in step 905 the result is positive, then in step 910 is tested if N1 is equal or less than N2. If not, then in step 911 a number $F_r(N1, N2)$ of times the entity in position N1 is connected to the entity in position N2 or the entity in position N2 is connected to the entity in position N1 in the M entity networks is computed, and steps 907 and 908 are executed. If the result of the test in step 910 is positive, then steps 906, 907, 908 and 911 are left out and the number N1 is decremented by 1 in step 909 immediately. In step 912 is tested if N1 is equal to zero. If not, then the steps 905 to 912 are repeated. If yes, then the number N2 is decremented by 1 in step 913. In step 914 is tested if N2 is equal to zero. If not, then the steps 905 to 914 are repeated. If yes, then in step 915 the list of pairs of entities of the established entity networks are ordered with respect to ratios. In step 916, the ordered list of pairs of entities of the established entity network with the ratios is outputted.

In figure 10, a procedure 1000 for determination of the threshold frequency ratio and of the final list of pairs of entities in causal relationship with the established entity network is depicted. In step 1001, M entity networks, the established entity network and information about if the analysis of the relationship is bidirectional are provided as input data. In step 1002, the established entity network relationship analysis procedure 900, as described with regard to figure

9, is called, providing a list of pairs of entities ordered by frequency ratios. In step 1003, ROC curves are computed varying the threshold frequency ratio. In step 1004, Precision-Recall curves are computed, varying the threshold frequency ratio. In step 1005 the curves are outputted. In step 1006 a threshold ratio is provided as input data. In step 1007 the list of pairs of entities whose frequency ratio is above the threshold is computed. In step 1008, the list of pairs of entities whose ratio is above the threshold is outputted.

In figure 11, an overall causal relationships discovery procedure 1100 is depicted. In step 1101, an initial set of entities, an established entity network, a subset size "s", a number of iterations $N_{iter}$ and expression data thereof are provided as input data. In step 1102 the application procedure 500 as described with regard to figure 5, is called. In step 1103, information if the analysis of the relationships is bidirectional is provided as input data. In step 1104, the threshold frequency ratio and the final list of pairs of entity in causal relationships within the established entity network are determined according to the procedure 1000 as depicted in figure 10. In step 1105, the list of pairs of entities whose ratio is above the threshold is outputted.

The overall causal relationships discovery procedure 1100, thus, comprises the application procedure 500 and the procedure 1000 for the determination of the threshold frequency ratio and of the final list of pairs of entities in causal relationship within the established entity network that exploits the established entity network relationships analysis procedure 900.

In figure 12, an internal-external relationships analysis procedure 1200 is depicted. In step 1201, the M entity networks, the established entity network, a list of external entities and information about if the analysis of the relationships is bidirectional are provided as input data. In step 1202, the list of the internal entities of the established entity network is computed. In step 1203, a number N1 of internal entities of the established entity network is computed. In step 1204, a number N2 as dimension of the list of external entities is computed. In step 1205, an initially empty list of pairs of entities is initialized. In step 1206 is tested if the analysis of the relationships is bidirectional. If not, then in step 1207 a number $F_r(N1, N2)$ of times that the internal entity in position N1 is connected to the external entity in position N2 in the M entity networks is computed. In step 1208 a ratio $r(N1, N2) = F_r(N1, N2)/M$ is computed. In step 1209, the pair (N1, N2) with the ratio r(N1, N2) is inserted in the list of pairs. In step 1210, the number N1 is decremented by 1. If the result of the test in step 1206 is positive, then in step 1211 is tested if N1 is equal or less than N2. If not, then in step 1212 a number $F_r(N1, N2)$ of times the internal entity in position N1 is connected to the external entity in position N2 or the external entity in position N2 is connected to the internal entity in position N1 in the M entity networks is computed and 1208 to 1210 are executed. If the result of the test in step 1211 is positive, then steps 1207 to 1209 and 1212 are left out and step 1210 is executed decrementing the number N1 by 1. In step 1213 is tested if the number N1 is equal to zero. If not, then the steps 1206 to 1213 are repeated. If yes, then in step 1214 the number N2 is decremented by 1. In step 1215 is tested if the number N2 is equal to zero. If not, then the steps 1206 to 1215 are repeated. If yes, then in step 1216 the list of internal-external entity pairs is ordered with respect to ratios. In step 1217, the ordered list of pairs of internal-external entities is outputted. Figure 13 shows a procedure 1300 for the determination of the threshold frequency ratio and of the final list of external-internal pairs of entities in causal relationship. In step 1301, the M entity networks, the established entity networks and a list of external entities are provided as input data. Furthermore is provided information about if the analysis of the relationships is bidirectional. In step 1302, a number of replicas $N_{rep}$ and a dimension d of the established entity networks subsets is provided as input data. In step 1303, a set D of d entities from the established entity network is randomly selected. In step 1304, an established entity sub-network with the entities in the set D and the edges that are also in the established entity network is built. In step 1305 the internal-external relationships analysis procedure 1200, as described with regard to figure 12, is called, providing a list of internal-external pairs of entities ordered by frequency ratios. Internal and external entities are to be understood with regard to the sub-network of an established entity network. In step 1306, a number of replicas $N_{rep}$ is decremented by 1. In step 1307 is tested if $N_{rep}$ is equal to zero. If not, then the steps 1303 to 1307 are repeated. If yes, then in step 1308 ROC curves varying the threshold frequency ratio are computed. In step 1309 Precision-Recall curves varying the threshold frequency are computed. In step 1310 the curves are outputted. In step 1311, a threshold ratio is provided as input data. In step 1312 the internal/external relationship analysis procedure 1200, as described with regards to figure 12, is called with the established entity network as input data providing a list of internal-external pairs of entities sorted by frequency ratios. In step 1313, the list of entities whose frequency ratio is above the threshold is computed. In step 1314, the list of pairs of entities, whose frequency is above the threshold is outputted.

In figure 14, an overall network expansion and internal-external causal relationships discovery procedure 1400 is depicted. In step 1401, a list of entities, an established entity network, a subset size "s", a number of iterations $N_{iter}$ and expression data thereof are provided as input data. In step 1402, the application procedure 500, as described with regard to figure 5, is called. In step 1403, a number of replicas $N_{rep}$ and a dimension d of the established set of entities is provided as input data. In step 1404 the threshold frequency ratio and the final list of entities that expand the network is determined according to procedure 700 as described with regard to figure 7. In step 1405, a list of entities whose ratio is above the threshold is outputted. In step 1406, information if the analysis of the relationships is bidirectional is provided as input data. In step 1407 a threshold frequency ratio and the final list of internal-external pairs of entities in causal

relationship are determined by calling the procedure 1300, as described with regard to figure 13. In step 1408, the list of internal-external pairs of entities whose ratio is above the threshold is outputted.

The overall network expansion and internal-external causal relationships discovery procedure 1400, thus, comprises the very same components of the overall network expansion procedure 800 and the procedure 1300 for the determination of the threshold frequency ratio and of the final list of internal-external pairs of entities in causal relationship that exploits the internal-external relationships analysis procedure 1200.

EXAMPLES

Performance Evaluation

This section deals with the evaluation of the network expansion procedure presented in the previous section. Next section is devoted to the presentation of the results.

Preliminary E*valuation 1:* in silico data vs in vivo data

The aim of this preliminary evaluation is to understand whether in silico or in vivo data have to be used for the evaluation of the PC-IM. In silico data are generated from mathematical equations, an example being DREAM data [Marbach et al., 2009] which provide plausible biological Gold Standard networks and the corresponding simulated gene expression data. We had reasons to believe that the PC algorithm that checks conditional independence between genes could be underrated by in silico data. To test this hypothesis we compared the performance using in vivo data (real gene expression data available in public databases) on the same GRN of the in silico data.

We applied PC and ARACNE algorithms to the time series data of the challenge 2 of DREAM4. In the challenge 2 there are simulated data for two different organisms (Escherichia coli and Saccharomyces cerevisiae) and for each organism there are GRNs with two different size (10 and 100 genes). In Table 1 the names of the GRNs for each organisms and for each size of the GRNs are reported.

Table 1 the GRN name identifies only the organism and not the size of GRN. In fact: rep1 and rep2 are the names for the GRN of the Escherichia coli. rep3, rep4 and rep5 are the GRN-name for the Saccharomyces cerevisiae. The same names are given to GRN of different size

(Table 1)

| GRN Size (nr genes) | Escherichia coli | Saccharomyces cerevisiae |
|---|---|---|
| 10 | rep1; rep2 | rep3; rep4; rep5 |
| 100 | rep1; rep2 | rep3; rep4; rep5 |

Algorithm performances have been evaluated by considering the PPV and Se values of the inferred GRN:

$$PPV = TP/(TP+FP) \quad (4)$$

$$Se = TP/(TP+FN) \quad (5)$$

where TP, FP, FN, FP are computed with respect to the known GRN.

Comparison of the results obtained using either in silico or in vivo data was carried out only with the Saccharomyces cerevisiae datasets. In particular two analyses were performed with in vivo data. In the first analysis we have considered the three GRNs (rep3, rep4 and rep5) for each size (10 and 100 genes). As expression data we used all the data available in the public database M3D (http://m3d.bu.edu/norm/). In the second analysis we have selected only the GRN rep3 with size 10 and in vivo data found at the GEO DataSet specific for yeast (http://www.ncbi.nlm.nih.gov/gds?term=saccharomyces). In this case we have considered only the expression data collected during stress experiments and therefore possibly related with the GRN under study.

*Preliminary Evaluation 2: PC vs ARACNE*

The aim of the second preliminary evaluation is to compare the performance of PC and ARACNE in the task of expansion of an LGN. This comparison is an essential step in order to decide whether our algorithm will use the PC

algorithm or another commonly used algorithm (ARACNE) to expand an LGN (step2). The comparison is done using in vivo data and a known GRN.

Gene Expression Data: observational data used for testing the algorithms were selected from the Arabidopsis microarray expression data available in the Plex database [http://www.plexdb.org]. They are 393 hybridization experiments of the Arabidopsis thaliana GeneChip® Arabidopsis ATH1 Genome Array. The GeneChip® ATH1, designed in collaboration with TIGR, contains more than 22,500 probe sets representing approximately 24,000 genes. All the data have been downloaded from the same database (Plexdb) in order to manipulate data which have been normalized in the same manner.

Gene Regulatory Network: the known GRN is the flower organ specification gene regulatory network (FOS-GRN) of the model plant Arabidopsis thaliana. In recent years the FOS-GRN has been characterized and validated in vivo by the use of specific mutants [Espinosa-Soto et al., 2004]. The FOS-GRN encompasses 15 genes linked by 54 causal relationships [Sanchez-Corrales et al., 2010]. The FOS-GRN will be the LGN.

Tiles Creation: subsets of genes with size 50, 100, 200, 500 and 1000 are randomly chosen among the ATH1 genes For each size, five different subsets were considered.. Each of them includes the 15 genes of the FOS-GRN plus 35, 85, 185, 485 or 985 additional genes randomly selected from the GeneChip® Arabidopsis ATH1 Genome Array.

subLGNs Generation: subLGNs with different sizes (3, 5 and 10 genes) were created. The subLGNs derive from the LGN and were obtained extracting randomly the genes of the FOS-GRN. For each subLGN the genes of the LGN are divided in two categories: INTRA and EXTRA genes (see step4 of the PC-IM, paragraph 2.1). For each size of subLGN we repeated 100 times the run. The evaluation criteria are PPV (Equation 1), Se (Equation 2), FPR (Equation 3), Number of total genes (Equation 6) and Delta (Equation 7):

$$\text{Number of total genes} = (TPintra+FPintra+FNintra+TNintra)+(TPextra+FPextra+FNextra+TNextra) \quad (6)$$

$$\text{Delta} = (TPintra+FPintra+FNintra+TNintra)-(TPextra+FPextra+FNextra+TNextra) \quad (7)$$

Evaluation

The PC-IM performance was evaluated using in vivo gene expression data (see the "Gene Expression Data" above) and a known LGN (see the "Gene Regulatory Network" above) of Arabidopsis thaliana. The aim was to give a first evaluation of the performance and in particular to study the effect of the size of the tiles (t) and of the number of iterations (i). Moreover we wanted to test the method in case the input is a GRN with no biological foundation. For this reason we compared the performance of the LGN expansion when we use the known LGN versus that of a random LGN. Overall the evaluation experiments are three:

Effect of the tile size (t)
Effect of the number of the iterations (i)
Known LGN vs. random LGN

Effect of the tile size (t)

This evaluation experiment investigates the influence of the size of the tiles on the PC-IM output in order to identify which value gives the best performance.

We run PC-IM with tiles of different size: 50, 100, 200, 500 and 1000 genes. Tiles generation is carried out as described above. The number of iteration (i) is 100 and the size of the subLGN (d) is 10 (see subLGN generation above). The best t is the one with the maximum value of AUC and the minimum value of dmin.

Effect of the number of iterations (i)

A key characteristic of the proposed method is iteration, namely PC-IM repeats several times the division of the whole genome into tiles. In this evaluation experiment, we study the effect of different values of the number of iterations (i) on the final PC-IM results.

Nine different values of i are analyzed: 1, 5, 10, 15, 20, 25, 50, 75 and 100. The subLGN dimension d is 10

and the size of the tiles (t) is 1000. As for the previous analysis, we select the value of i whose corresponding frequency is the best compromise between maximum AUC and minimum dmin.

Known LGN vs Random LGN

We test the robustness of our system when a non-real GRN is provided as input. To this aim we compare in terms of PPV, Se, dmin of the PR curve and cut-off frequency the results when the input is a real LGN against an input LGN randomly selected from the genes of the input dataset (Random LGN). The real LGN is the FOS-GRN and the Random LGN has the same size (number of genes) and the same number of relationships of the FOS-GRN (54 relationships) but the genes are randomly sampled from the the genes of the ATH1 GeneChip®. and the relationships are also randomly assigned. For the same Random LGN three different combination of relationships are tested. For this test we set i = 100 and t = 1000.

Biological Validation

In this evaluation we apply PC-IM directly to the FOS-GRN in order to expand it. Results are evaluated by comparison with the available literature. PC-IM is run using the following parameters: t =1000, i=100, LGN= 15 genes of the FOS-GRN and d=10.

By doing a bibliographic search of the genes provided in output by PC-IM, we could identify four classes:

Class 1 = genes related to flowering
Class 2 = genes not directly related to flowering, but related to Class 1
Class 3 = genes unrelated to flowering
Class 4 = genes for which no references were found

Results

Preliminary Evaluations 1 and 2

The purposes of the preliminary evaluations are (1) to understand which type of gene expression data (in silico or in vivo) is more reliable for the evaluation and (2) which algorithm, PC or ARANCE, is the most effective to be used in our GRN expansion approach.

To this aim both types of data (in silico or in vivo) have been firstly tested with PC and ARACNE algorithms. The in silico data derived from the DREAM project [Marbach et al., 2009] and the performances of the algorithms are reported in Table 2 and 3. When applied to in silico gene expression data ARACNE showed a greater precision and PC-algorithm a greater sensitivity. However, the algorithm performances changed when we used the same GRN with in vivo data (Table 4 and 5 of the Supplementary material). In fact, when all the gene expression data available on the M3d website (http://m3d.bu.edu/norm/) is used the sensitivity of both algorithms is comparable to the value obtained with in-silico data, whereas the precision of PC improves with respect to the precision of ARACNE (Table 4). Further, this gain in precision is more evident when the in vivo data are inherent to the LGN (Table 5).

The performances of PC-algorithm and ARACNE in the LGN expansion task are showed in Figure 17.

For this test 3 different values of the size of the subLGN (3, 5 and 10 genes) and 5 different values of the size of the dimension of the tiles (50, 100, 200, 500 and 1000 genes) were assessed. For each different size of the tiles and for each size of the subLGN, 4 repetitions and 100 repetitions were carried out, respectively.

Figure 17-tot nodes curves shows that the total number of genes (computed as in equation 6) found by the algorithms. With 50 genes tiles, PC and ARACNE found a comparable number of genes, whereas with the other tile sizes, ARACNE selected much more nodes with respect to PC.

Figure 17-Delta curve reports the Delta criterion (computed as in equation 7) to compare these two algorithms. The best performance in terms of Delta values is reached when the Delta value is positive or less negative, because this means that the algorithm finds more INTRA nodes than EXTRA nodes. To this regard PC shows a Delta value close to zero for all the tile sizes and in general is less negative than ARACNE. The difference of the Delta values is more evident with tiles of 500 genes when ARACNE displayed the most negative Delta value. Figure 17-PPV curve and

Figure 17-Se curve report the values of PPV (computed as in equation 1) and the Sensitivity (computed as in equation 2). PPV of PC is greater than that of ARACNE, except in the case of tiles with 1000 genes (Figura 2C). The Sensitivity of ARACNE is greater of the PC Sensitivity (Figure 2D), except in the following cases:

with subLGN of 10 genes and tiles with 200 and 1000 genes, PC and ARACNE have similar Sensitivity values;
with subLGN of 5 genes and tiles with 500 genes, PC and ARACNE have similar Sensitivity values; with subLGN

of 10 genes and tiles with 500 genes, PC reaches a greater Sensitivity with respect to the Sensitivity of ARACNE.

The selection of the PC is made for two reasons. (1) The preliminary evaluations show that it improves its performance with real gene expression data. Whereas ARACNE does not seem to be influenced by the different type of data. (2) At the moment there are not in vivo systems such that they can easily support the contemporary manipulation of a big number of genes, posing a practical limit to the number of gene that are reasonable to validate. Furthermore none of the algorithms, on the in vivo data, has a a PPV value of 100%. This mean that it is better to choose an algorithm with a good value of PVV that it can find a low number of genes that expand a specific LGN.

Evaluation

Selection of the tile size (t)

In order to evaluate the performance of PC-IM during the expansion of the LGN, ROC and PR curves were built. 5 different values of the size of the tiles (50, 100, 200, 500 and 1000 genes) with no replicates and a LGN of 10 genes were considered. The run was iterated 100 times.

Initially, we have identified the best size of the tiles. As reported in Figures 15 and 16 the AUC value of ROC curve and dmin value of PR-curve were optimal when tiles of 200, 500 and 1000 genes were considered for the expansion of the LGN (Table 6).

Table 6: Values of AUC and d_min depending on the size, namely the number of genes, of the tiles.

| Size of the tile | 50 | 100 | 200 | 500 | 1000 |
|---|---|---|---|---|---|
| AUC | 0.662 | 0.663 | 0.710 | 0.706 | 0.700 |
| dmin | 0.481 | 0.496 | 0.547 | 0.560 | 0.562 |

Selection of the iteration number (i)

Once identified the size of the tiles we have evaluated the influence of the number of iterations on the performances of PC-IM. We have tested 9 different numbers of the iterations (1, 5, 10, 15, 20, 25, 50, 75 and 100) using 1000 genes as tile size. The results are shown in Figures 18 and 19. The best performance (low AUC-value (ROC curve) and high dmin value (PR-curve)) were obtained with 100 iterations (Table 7).

Table 7: Values of AUC and dmin depending on the number of iterations and with fixed tile size (1000 genes).

| Nr of iter. | 1 | 5 | 10 | 15 | 20 | 25 | 50 | 75 | 100 |
|---|---|---|---|---|---|---|---|---|---|
| AUC | 0.730 | 0.747 | 0.727 | 0.683 | 0.683 | 0.719 | 0.713 | 0.683 | 0.700 |
| dmin | 0.834 | 0.676 | 0.738 | 0.723 | 0.724 | 0.611 | 0.573 | 0.533 | 0.562 |

Known LGN vs Random LGN

The previous tests have demonstrated that the best value of the size of the tile and of the number of iterations were 1000 genes and 100 iterations, respectively. Before validating the genes identified in the expansion of FOS-GRN, we tested whether the performances (PPV, Se) observed when starting from the FOS-GRN as LGN are different from those obtained when starting from a Random LGN. Table 8 shows that PC-IM reaches better PPV, Sensititivity value, and smaller dmin of the PR curve in the first case. Also the frequency which provides the best performances is very different, the frequency computed for the FOS-GRN being 10 times greater than the one of the Random LGN. This shows that the method is robust and that the way of assessing PPV and Sensitivity can be a way of diagnosing the presence of faulty inputs as a random LGN is.

Table 8: Performance of PC-IM in expanding a real LGN (FOS-GRN) or a not-existing LGN (Random LGN). PPV max and Se max are the maximun value obtained of PPV and Se, respectively; dmin is the minimal distance of the PR curve and freq% indicates the value of frequency (step 3 of the PC-IM) at which PPV max, Se max and dmin were obtained.

| | PPV max | Se Max | dmin | ($PR_{freq}$ % |
|---|---|---|---|---|
| FOS-GRN | 0.82230 | 0.46700 | 0.56200 | 62 |

(continued)

| | PPV max | Se Max | dmin | (PR$_{freq}$ % |
|---|---|---|---|---|
| Random LGN | 0.00016 | 0.09133 | 1.35100 | 6 |

Biological Validation

Applying the PC-IM on the FOS-GRN (1000 genes as tile size and 100 iterations) we have obtained 314 genes that expand this specific LGN. The PPV and Sensitivity are 82% and 46 % respectively.

Validation of the PC-IM results were done by bibliographic search. The aim is to understand if the estimated PPV (82 %) which is calculated within the LGN can be a reliable estimation of the PPV of the final expanded LGN. The problem is that very often and especially in the case of complex organisms, not all the genes of the organism have been identified and functionally annotated. For this reason we divided the bibliographic results in 4 classes as described in Table 9. The bibliographic search has shown that 13% of the genes were not related with the flowering process (Class 3), but 36% of the genes are related, directly or indirectly (Class 1 and 2) with the flowering. For the remaining 50% of the 314 genes we did not find any references on their function. This implies that we cannot validate all 314 genes with the bibliographic search. In fact the genes of Class 4 could or could not be involved in the flowering and further biological investigation is required in order to validate them. However, if we consider the genes in Class3 as true negatives and we do not consider the genes in Class 4 we reach a putative precision of 74%.

Table 9: Class 1 = genes related with flowering; Class 2 = genes not directly related with flowering, but with Class 1; Class 3 = genes Unrelated with flowering; Class 4 = genes with no references

| | Class 1 | Class 2 | Class 3 | Class 4 |
|---|---|---|---|---|
| Nrgenes | 54 | 60 | 41 | 159 |

References

Marbach,D. and Schaffter,T. and Mattiussi,C. and Floreano,D. (2009); Generating realistic in silico gene networks for performance assessment of reverse engineering methods; J Comput Biol. Feb;16(2):229-39;

Wang, C. and Marshall, A. and Zhang, D. and Wilson, Z.A. (2012); ANAP: An Integrated Knowledge Base for Arabidopsis Protein Interaction Network Analysis; Plant physiology, Apr;158(4):1523-33;

Sanchez-Corrales,Y.E. and Alvarez-Buylla,E.R. and Mendoza,L. (2010); The Arabidopsis thaliana flower organ specification gene regulatory network determines a robust differentiation process; Journal of theoretical biology 7;264(3):971-83.

**Table 2:** DREAM4, challenge 2, time series, *Escherichia coli* transcriptional regulatory network (rep1 and rep2) and two different sample size with 10 genes (size 10) and 100 genes (size 100).

| size | **algorithm** | **TP** | **FP** | **FN** | **PPV** | **Se** |
|---|---|---|---|---|---|---|
| 10 (rep1) | PC | 10 | 5 | 5 | 0.667 | **0.667** |
| | ARACNE | 3 | 0 | 12 | **1.000** | 0.200 |
| 10 (rep2) | PC | 5 | 8 | 11 | 0.385 | **0.313** |
| | ARACNE | 3 | 1 | 13 | **0.750** | 0.188 |
| 100 (rep1) | PC | 28 | 156 | 148 | 0.152 | **0.159** |
| | ARACNE | 23 | 116 | 153 | **0.165** | 0.131 |
| 100 (rep2) | PC | 14 | 156 | 235 | **0.082** | **0.056** |
| | ARACNE | 10 | 116 | 239 | 0.079 | 0.040 |

**Table 3:** DREAM4, challenge 2, time series, *Saccharomyces cerevisiae* transcriptional regulatory network (rep3, rep4 and rep5) and two different sample size with 10 genes (size 10) and 100 genes (size 100).

| size | algorithm | TP | FP | FN | PPV | Se |
|---|---|---|---|---|---|---|
| 10 (rep3) | PC | 6 | 5 | 9 | 0.545 | **0.400** |
| | ARACNE | 4 | 1 | 11 | **0.800** | 0.267 |
| 10 (rep4) | PC | 7 | 5 | 6 | 0.583 | **0.538** |
| | ARACNE | 5 | 0 | 8 | **1.000** | 0.385 |
| 10 (rep5) | PC | 10 | 5 | 2 | **0.667** | **0.833** |
| | ARACNE | 7 | 0 | 5 | 0.538 | 0.583 |
| 100 (rep3) | PC | 36 | 134 | 159 | 0.212 | 0.185 |
| | ARACNE | 37 | 89 | 158 | **0.294** | **0.190** |
| 100 (rep4) | PC | 30 | 163 | 181 | 0.155 | **0.142** |
| | ARACNE | 29 | 109 | 182 | **0.210** | 0.137 |
| 100 (rep5) | PC | 35 | 155 | 158 | 0.184 | **0.181** |
| | ARACNE | 33 | 100 | 160 | **0.248** | 0.171 |

**Table 4:** DREAM4, challenge 2, *Saccharomyces cerevisiae*. *In vivo* data (m3d gene expression data). Number of Experiments: 904.

| size | algorithm | TP | FP | FN | PPV | Se |
|---|---|---|---|---|---|---|
| 10 (rep3) | PC | 6 | 8 | 9 | 0.429 | **0.400** |
| | ARACNE | 3 | 2 | 12 | **0.600** | 0.200 |
| 10 (rep4) | PC | 3 | 11 | 10 | 0.214 | **0.231** |
| | ARACNE | 1 | 1 | 12 | **0.500** | 0.077 |
| 10 (rep5) | PC | 3 | 12 | 8 | 0.250 | **0.333** |
| | ARACNE | 3 | 2 | 9 | **0.600** | **0.250** |
| 100 (rep3) | PC | 14 | 128 | 181 | **0.099** | 0.072 |
| | ARACNE | 12 | 164 | 183 | 0.068 | 0.062 |
| 100 (rep4) | PC | 13 | 143 | 198 | 0.083 | 0.062 |
| | ARACNE | 17 | 131 | 194 | **0.115** | **0.081** |
| 100 (rep5) | PC | 1 | 155 | 192 | 0.006 | 0.005 |
| | ARACNE | 1 | 147 | 192 | **0.007** | 0.005 |

**Table 5:** DREAM4, challenge 2, *Saccharomyces cerevisiae*, rep3, size 10. *In vivo* data (GEO). (HS is Heat Schock gene observational expression data; Hypo means hypoxic gene observational expression data; Anaerobic is Anaerobic gene observational expression data; HS+ Hypo+ Anaerobic is sum of the gene observational data submitted before, n.d. means not determine)

| Name of experiments | **algorithm** | **TP** | **FP** | **FN** | **PPV** | **Se** |
|---|---|---|---|---|---|---|
| HS | PC | 4 | 9 | 11 | 0.308 | **0.267** |
| HS | ARACNE | 2 | 3 | 13 | **0.400** | 0.133 |
| Osmotic | PC | 4 | 1 | 11 | **0.800** | **0.267** |
| Osmotic | ARACNE | 1 | 1 | 14 | 0.500 | 0.067 |
| Anaerobic | PC | 4 | 5 | 11 | **0.444** | **0.268** |
| Anaerobic | ARACNE | 0 | 0 | 15 | n.d. | 0.000 |
| HS+Osmotic +Anaerobic | PC | 5 | 9 | 10 | 0.357 | **0.333** |
| HS+Osmotic +Anaerobic | ARACNE | 2 | 3 | 13 | **0.400** | 0.133 |

## Claims

1. A method for determining suitable entities for expanding an established causal molecular biological network, said method comprising:

    A) Providing an established causal molecular biological network, a candidate set of entities and abundance data thereof;
    B) Generating at least one intermediate expansion of said established causal molecular biological network with a defined part of the candidate set of entities by use of a computer program and by using the abundance data;
    C) Determining a measure of interrelationship for every entity of the candidate set of entities with respect to at least some of the entities of the established causal molecular biological network;
    D) Selecting entities out of the candidate set of entities based on the determined measure of interrelationship.

2. The method according to claim 1, wherein the candidate set of entities contains partial or whole genomes or partial or whole metagenomes.

3. The method according to any one of the preceding claims, wherein the measure of interrelationship is determined by calculating a quotient of a number of times that said entity has a direct or indirect binding with entities from the established causal molecular biological network and a number of appearances in the intermediate expansions.

4. A method for expanding an established causal molecular biological network, wherein entities from a candidate set of entities are selected according to a method according to any one of the preceding claims and wherein the selected entities are added to the established causal molecular biological network.

5. A method for determining significant causal relationships between entities of an established causal molecular biological network and candidate entities, said method comprising:

    A) Providing an established causal molecular biological network, a candidate set of entities and abundance data thereof;
    B) Generating at least one intermediate expansion of the said established causal molecular biological network or at least one intermediate expansion of a part of said established causal molecular biological network with a defined part of the candidate set of entities by use of a computer program and by using said abundance data;
    C) Determining a measure of interrelationship for every pair of entities, wherein one entity is from the candidate set of entities and a second entity is from said established causal molecular biological network or from the part

of said established causal molecular biological network;
D) Selecting pairs of entities based on the determined measure of interrelationship.

6. The method according to claim 5, wherein the candidate set of entities contains partial or whole genomes or partial or whole metagenomes and/or wherein a subset of the entities from the established causal molecular biological network forms the candidate set of entities or a fraction of the candidate set of entities and the remaining entities of the established causal molecular biological network form the part of the said established causal molecular biological network.

7. The method according to claim 5 or 6, wherein the measure of interrelationship is determined by counting the number of times the entities from the pair of entities are connected to each other in the intermediate expansions.

8. The method according to any one of the preceding claims, wherein said step B) comprises applying a causal network discovery algorithm to the entities.

9. The method according to any one of the preceding claims, wherein step B) comprises dividing the candidate set of entities into a defined number $N_{SUB}$ of subsets each having a defined number "s" of entities and establishing $N_{SUB}$ intermediate expansions.

10. The method according to any one of the preceding claims, wherein step B) is iteratively repeated for a number $N_{iter}$ of iterations.

11. The method according to any one of the preceding claims, wherein said selection in step D) comprises comparing the measure of interrelationship for every entity with a threshold value derived from a measure of interrelationship of entities from the established causal molecular biological network or comparing the measure of interrelationship for every pair of entities with a threshold value derived from a measure of interrelationship of pairs of entities from the established causal molecular biological network or from the part of said established causal molecular biological network.

12. The method according to claim 11, wherein the threshold value is determined by applying the steps A), B) and C) to a subset of entities from the established causal molecular biological network forming the candidate set of entities or the fraction of the candidate set of entities.

13. A method for finding candidate targets for a drug or for determining an impact of a drug or for detecting conspicuous cells or for crop breeding comprising performing the steps of a method according to any one of the preceding claims.

14. A computer program product directly loadable into the internal memory of a digital computer, comprising software code portions suitable for implementing one of the methods as described in any one of the preceding claims, when said product is run on a computer.

15. An apparatus for determining suitable entities for expanding an established causal molecular biological network, for expanding an established causal molecular biological network and/or for determining significant causal relationships between entities, the apparatus comprising:

- an input means for providing an established causal molecular biological network, a candidate set of entities and abundance data thereof;
- a unit for generating intermediate expansions of said established causal molecular biological network or of parts of said established causal molecular biological network with defined parts of the candidate set of entities by using said entity abundance data;
- a unit for determining a measure of interrelationship for entities or pairs of entities and
- a unit for selecting entities or pairs of entities based on the determined measure of interrelationship.

Figure 1

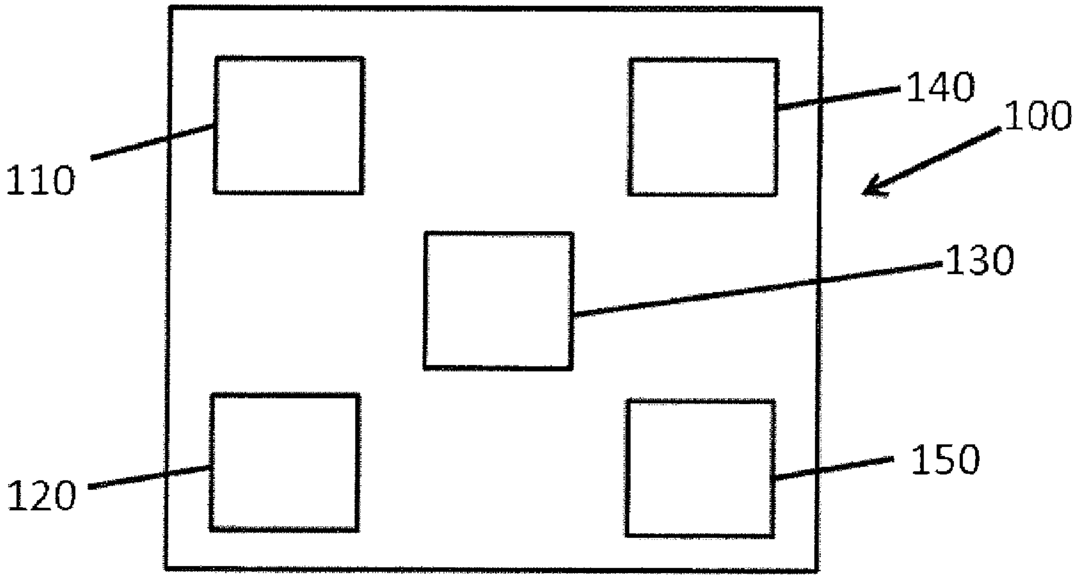

Figure 2

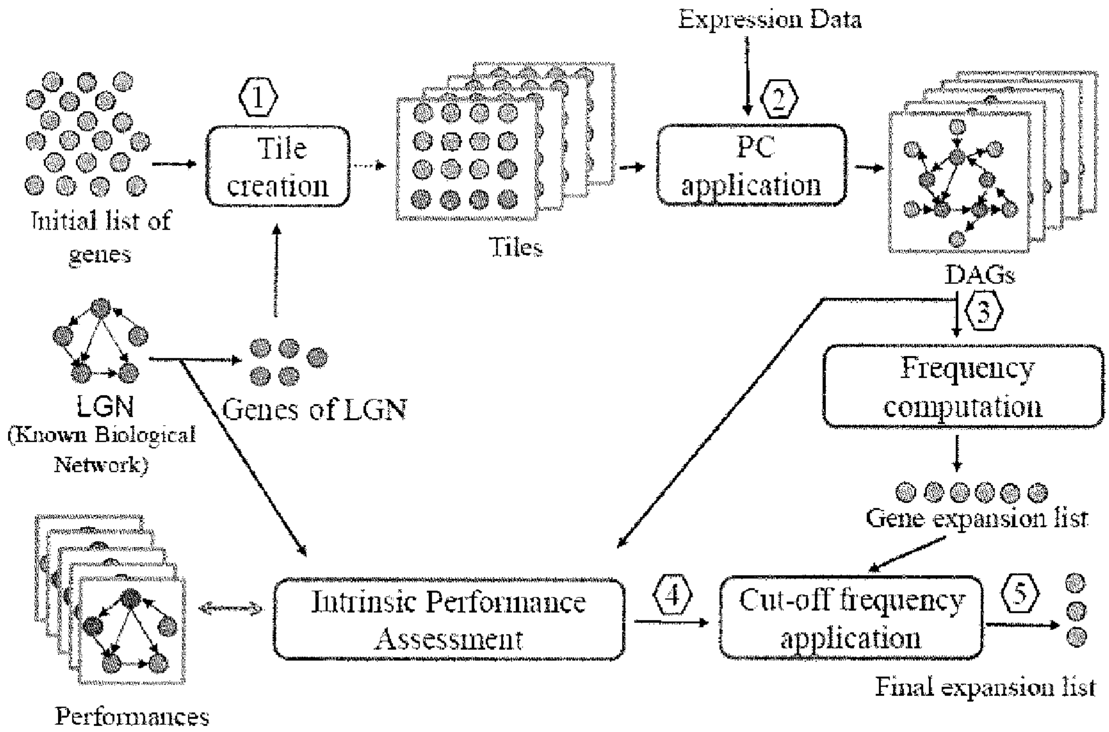
Expression Data
1
Tile creation
Initial list of genes
Tiles
2
PC application
DAGs
3
Frequency computation
LGN
(Known Biological Network)
Genes of LGN
Gene expansion list
Intrinsic Performance Assessment
4
Cut-off frequency application
5
Final expansion list
Performances

Figure 3

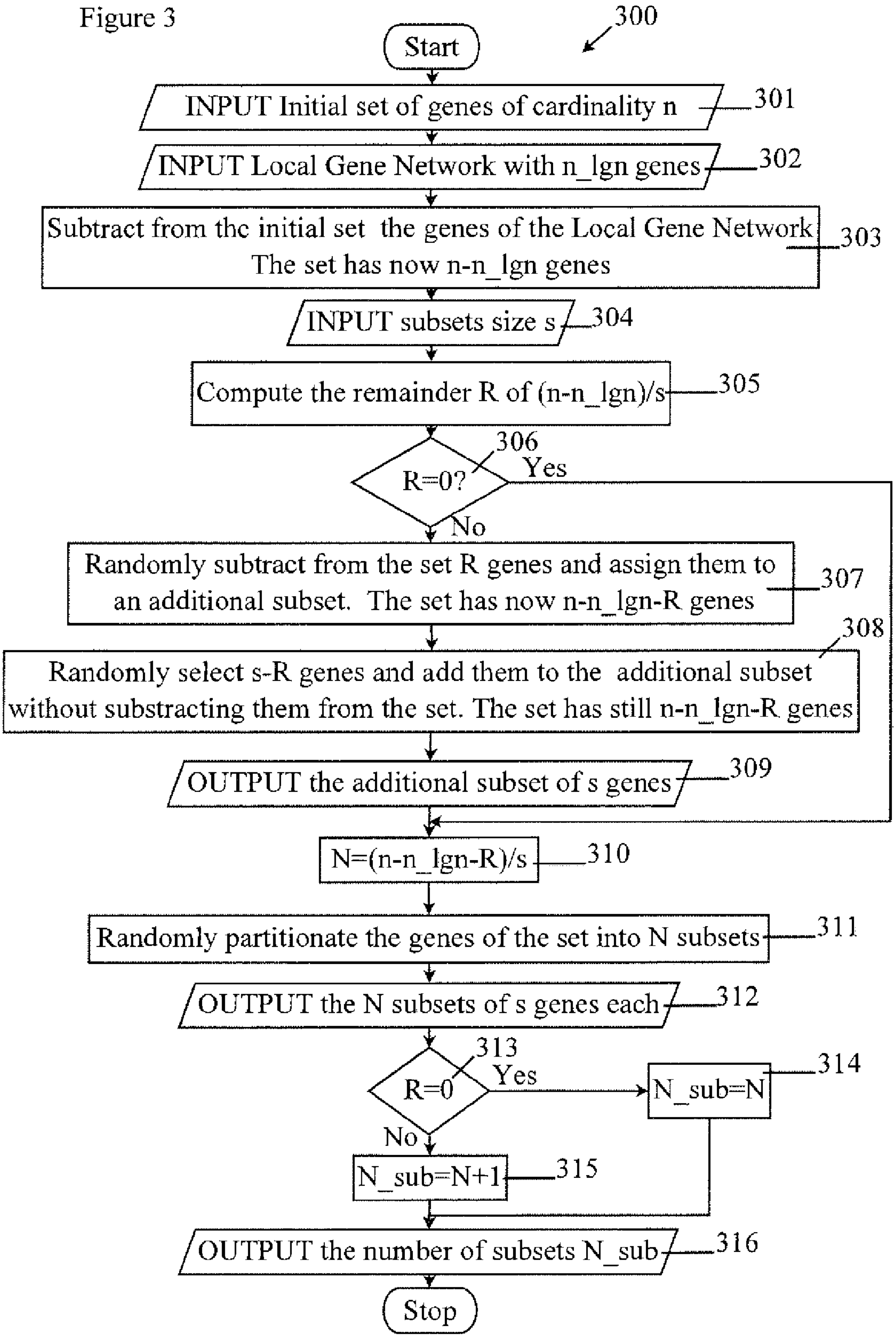
300
Start
INPUT Initial set of genes of cardinality n
301
INPUT Local Gene Network with n_lgn genes
302
Subtract from thc initial set the genes of the Local Gene Network
The set has now n-n_lgn genes
303
INPUT subsets size s
304
Compute the remainder R of (n-n_lgn)/s
305
306
R=0?
Yes
No
Randomly subtract from the set R genes and assign them to an additional subset. The set has now n-n_lgn-R genes
307
308
Randomly select s-R genes and add them to the additional subset without substracting them from the set. The set has still n-n_lgn-R genes
OUTPUT the additional subset of s genes
309
N=(n-n_lgn-R)/s
310
Randomly partitionate the genes of the set into N subsets
311
OUTPUT the N subsets of s genes each
312
313
R=0
Yes
N_sub=N
314
No
N_sub=N+1
315
OUTPUT the number of subsets N_sub
316
Stop

Figure 4

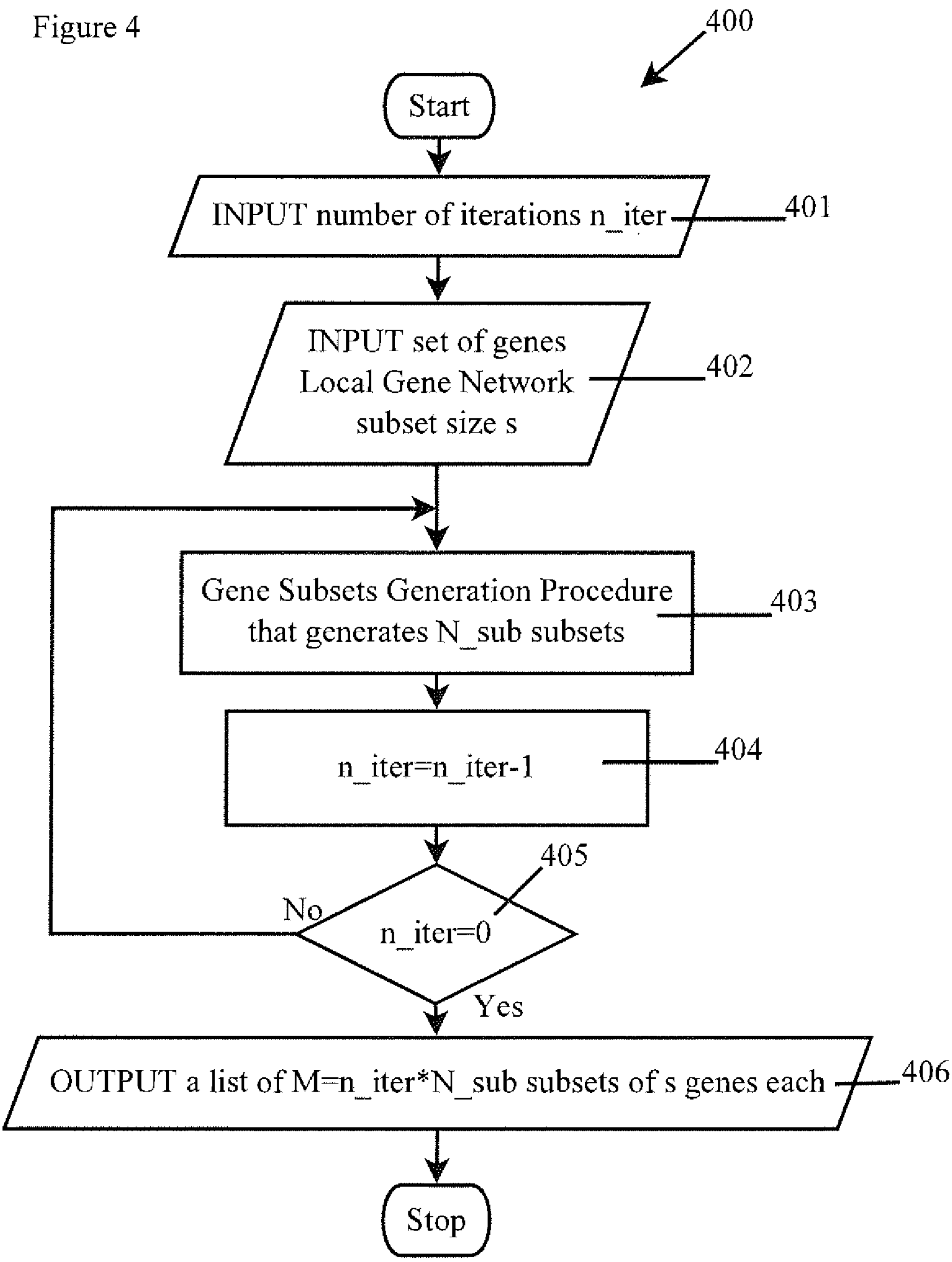
400
Start
INPUT number of iterations n_iter
401
INPUT set of genes
Local Gene Network
subset size s
402
Gene Subsets Generation Procedure
that generates N_sub subsets
403
n_iter=n_iter-1
404
405
No
n_iter=0
Yes
OUTPUT a list of M=n_iter*N_sub subsets of s genes each
406
Stop

Figure 5

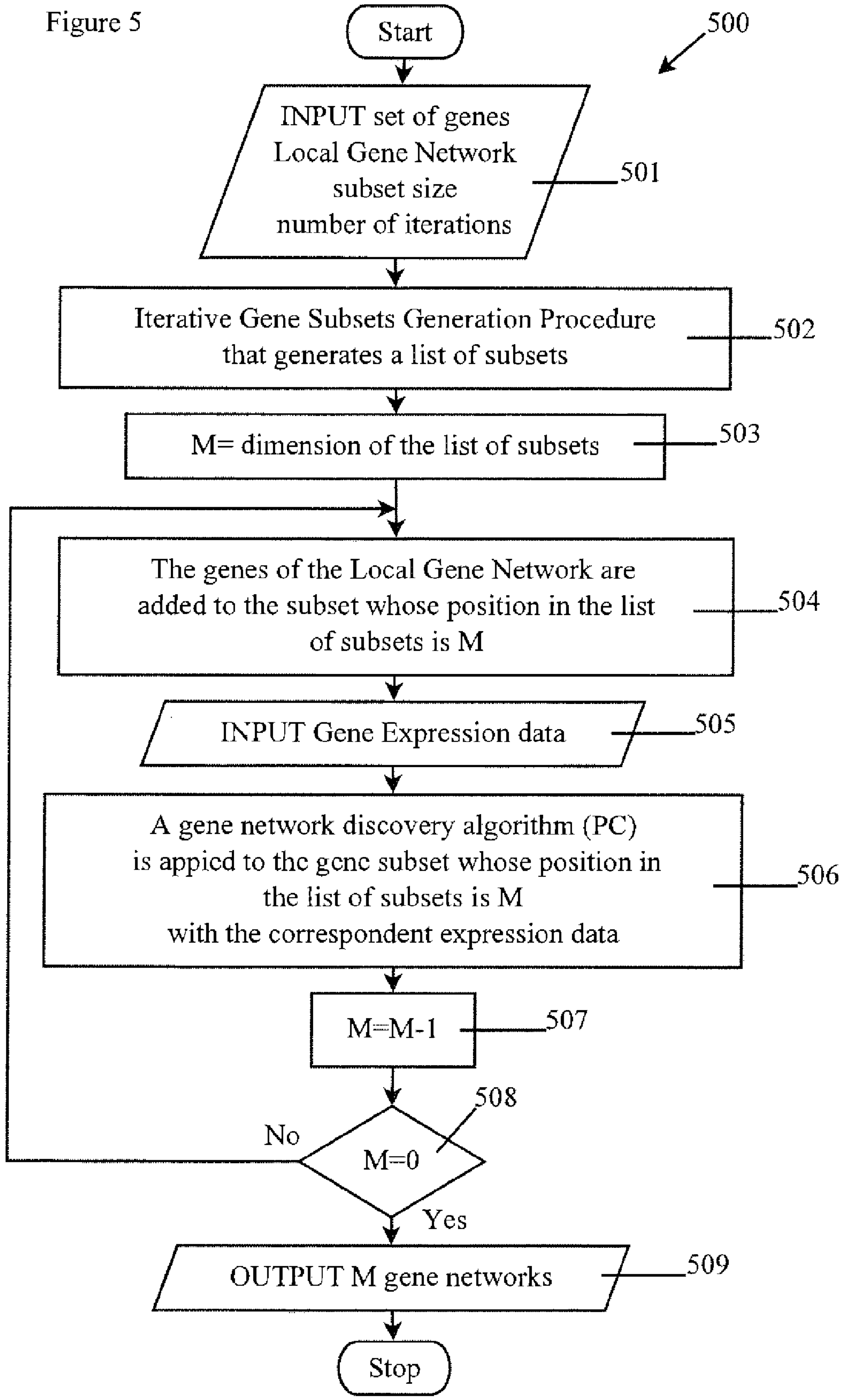

Start
500
INPUT set of genes
Local Gene Network
subset size
number of iterations
501
Iterative Gene Subsets Generation Procedure
that generates a list of subsets
502
M= dimension of the list of subsets
503
The genes of the Local Gene Network are
added to the subset whose position in the list
of subsets is M
504
INPUT Gene Expression data
505
A gene network discovery algorithm (PC)
is appicd to thc gcnc subset whose position in
the list of subsets is M
with the correspondent expression data
506
M=M-1
507
508
No
M=0
Yes
OUTPUT M gene networks
509
Stop

Figure 6

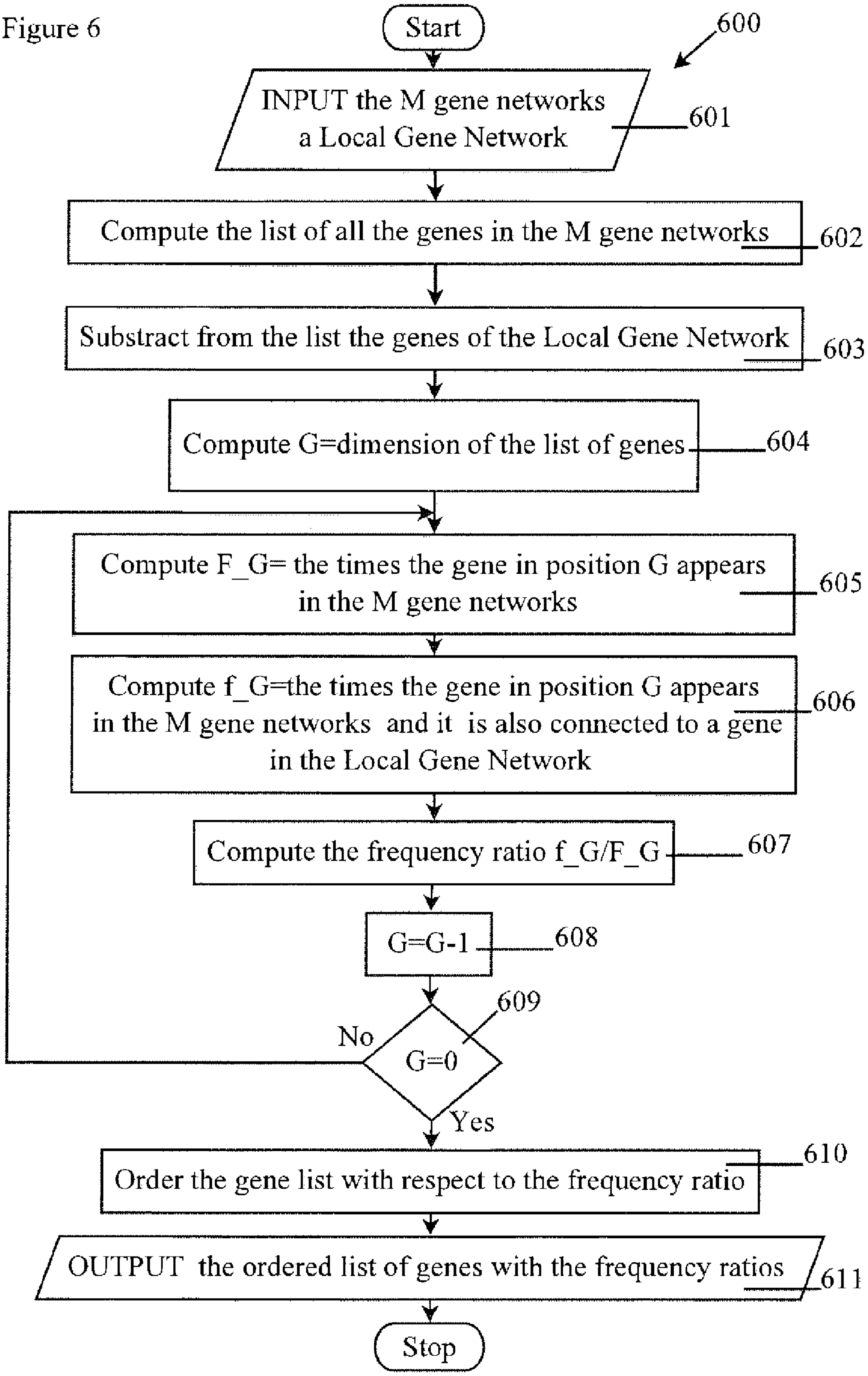

600
Start
INPUT the M gene networks a Local Gene Network
601
Compute the list of all the genes in the M gene networks
602
Substract from the list the genes of the Local Gene Network
603
Compute G=dimension of the list of genes
604
Compute F_G= the times the gene in position G appears in the M gene networks
605
Compute f_G=the times the gene in position G appears in the M gene networks and it is also connected to a gene in the Local Gene Network
606
Compute the frequency ratio f_G/F_G
607
G=G-1
608
609
No
G=0
Yes
Order the gene list with respect to the frequency ratio
610
OUTPUT the ordered list of genes with the frequency ratios
611
Stop

Figure 7

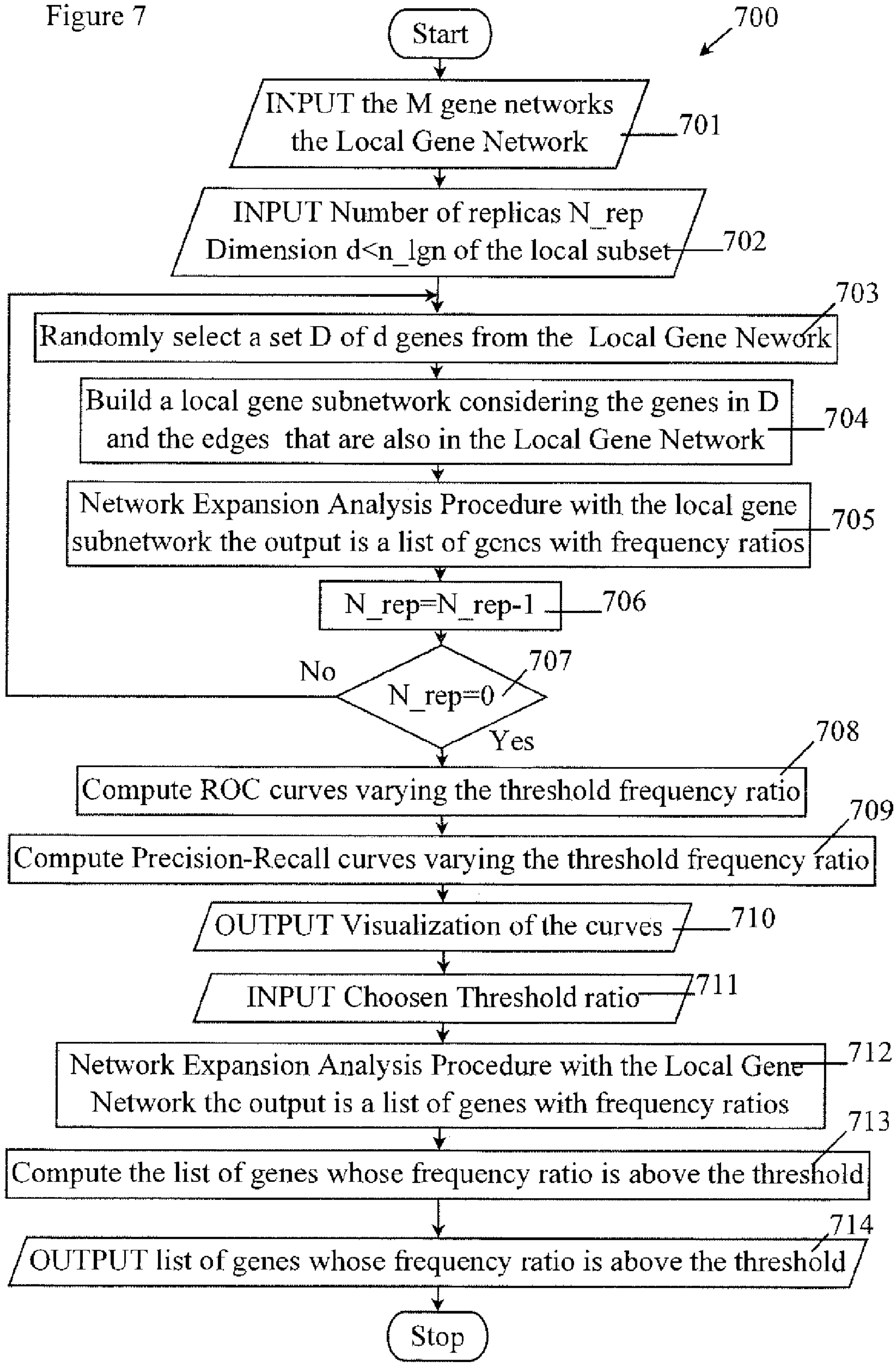
Start
700
INPUT the M gene networks the Local Gene Network
701
INPUT Number of replicas N_rep Dimension d<n_lgn of the local subset
702
703
Randomly select a set D of d genes from the Local Gene Nework
Build a local gene subnetwork considering the genes in D and the edges that are also in the Local Gene Network
704
Network Expansion Analysis Procedure with the local gene subnetwork the output is a list of genes with frequency ratios
705
N_rep=N_rep-1
706
No
N_rep=0
707
Yes
708
Compute ROC curves varying the threshold frequency ratio
709
Compute Precision-Recall curves varying the threshold frequency ratio
OUTPUT Visualization of the curves
710
INPUT Choosen Threshold ratio
711
Network Expansion Analysis Procedure with the Local Gene Network the output is a list of genes with frequency ratios
712
713
Compute the list of genes whose frequency ratio is above the threshold
714
OUTPUT list of genes whose frequency ratio is above the threshold
Stop

Figure 8

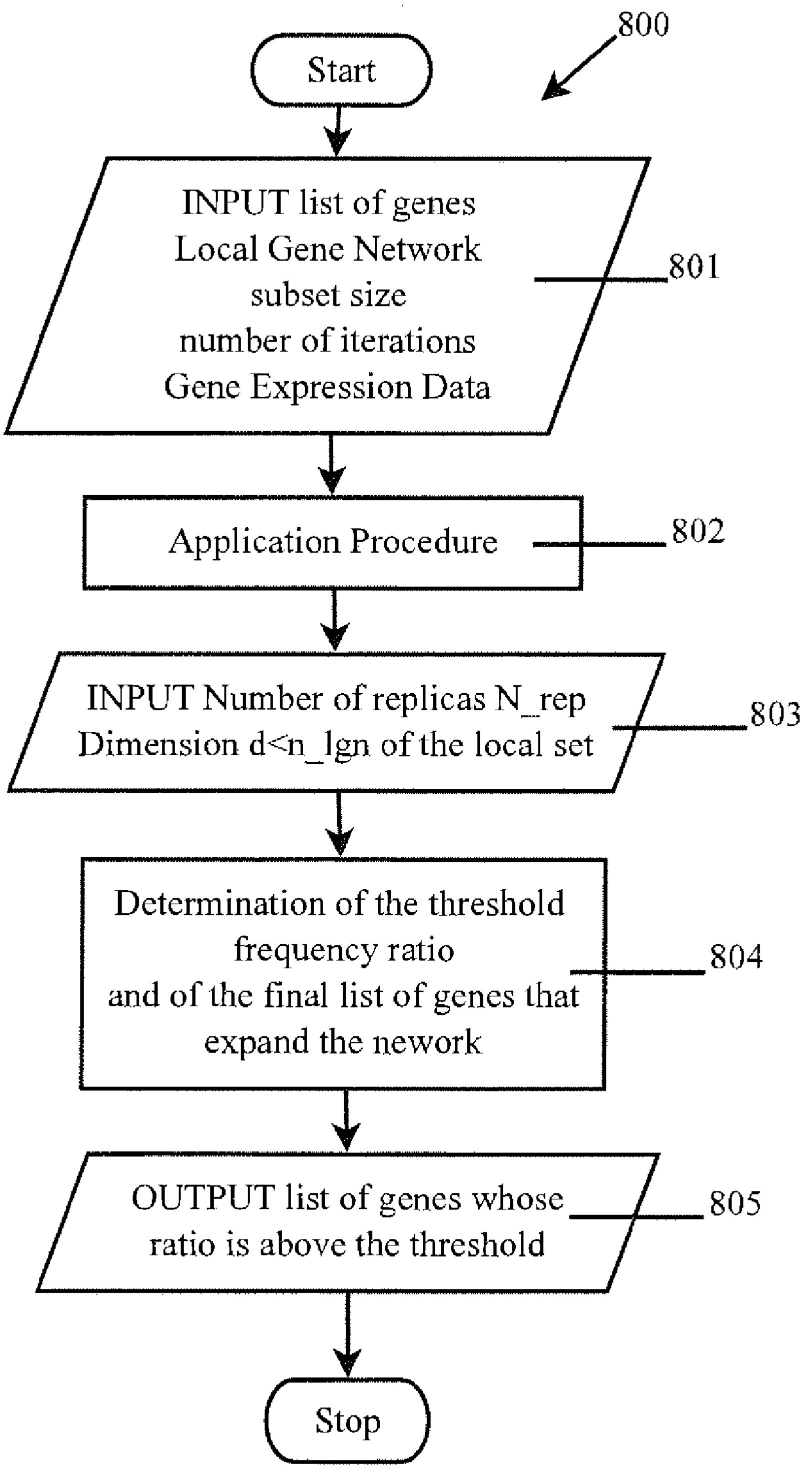
800
Start
INPUT list of genes
Local Gene Network
subset size
number of iterations
Gene Expression Data
801
Application Procedure
802
INPUT Number of replicas N_rep
Dimension d<n_lgn of the local set
803
Determination of the threshold
frequency ratio
and of the final list of genes that
expand the nework
804
OUTPUT list of genes whose
ratio is above the threshold
805
Stop

Figure 9

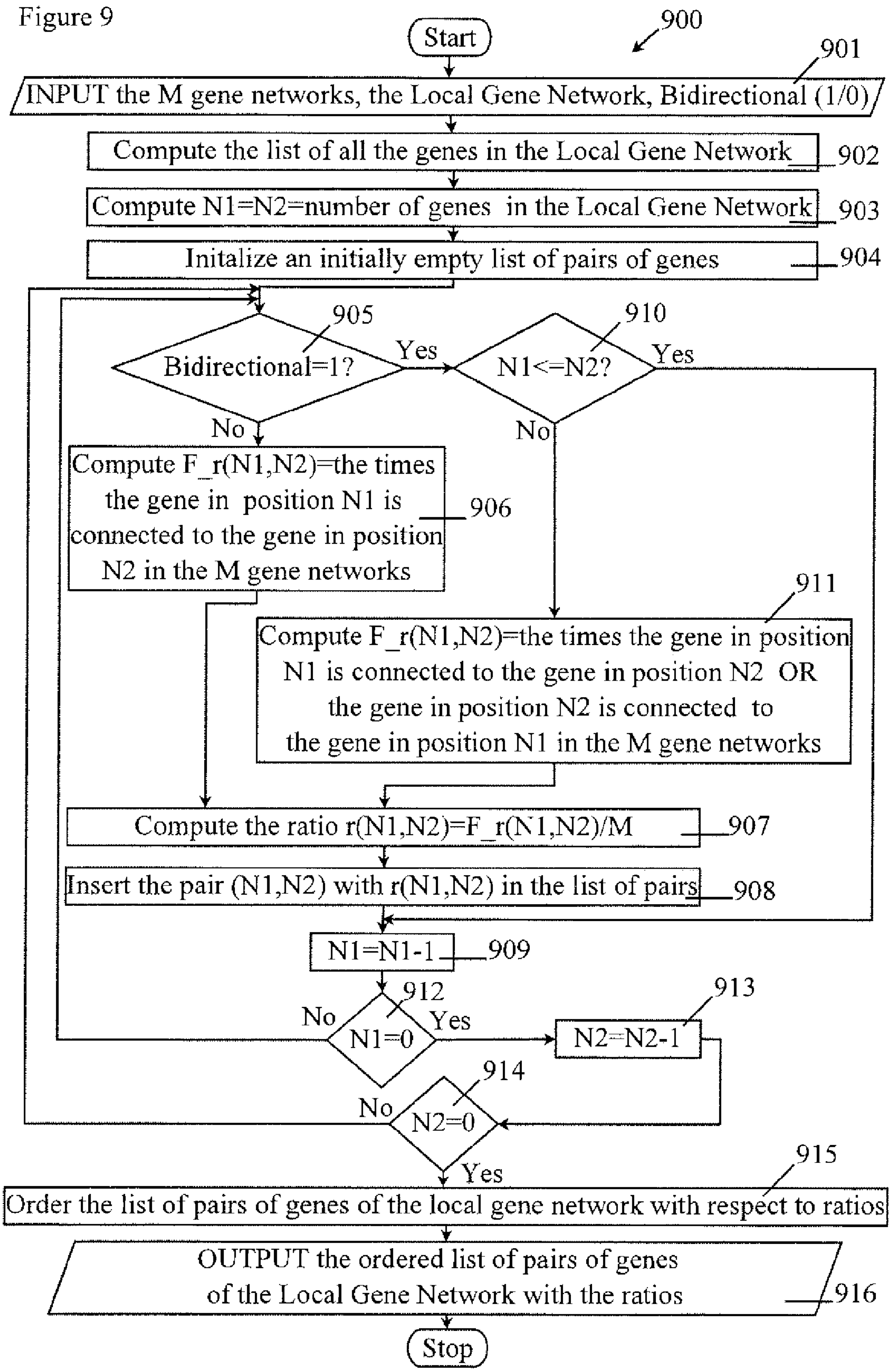
900
Start
901
INPUT the M gene networks, the Local Gene Network, Bidirectional (1/0)
Compute the list of all the genes in the Local Gene Network
902
Compute N1=N2=number of genes in the Local Gene Network
903
Initalize an initially empty list of pairs of genes
904
905
Bidirectional=1?
Yes
No
910
N1<=N2?
Yes
No
Compute F_r(N1,N2)=the times the gene in position N1 is connected to the gene in position N2 in the M gene networks
906
911
Compute F_r(N1,N2)=the times the gene in position N1 is connected to the gene in position N2 OR the gene in position N2 is connected to the gene in position N1 in the M gene networks
Compute the ratio r(N1,N2)=F_r(N1,N2)/M
907
Insert the pair (N1,N2) with r(N1,N2) in the list of pairs
908
N1=N1-1
909
912
No
N1=0
Yes
913
N2=N2-1
914
No
N2=0
Yes
915
Order the list of pairs of genes of the local gene network with respect to ratios
OUTPUT the ordered list of pairs of genes of the Local Gene Network with the ratios
916
Stop

Figure 10

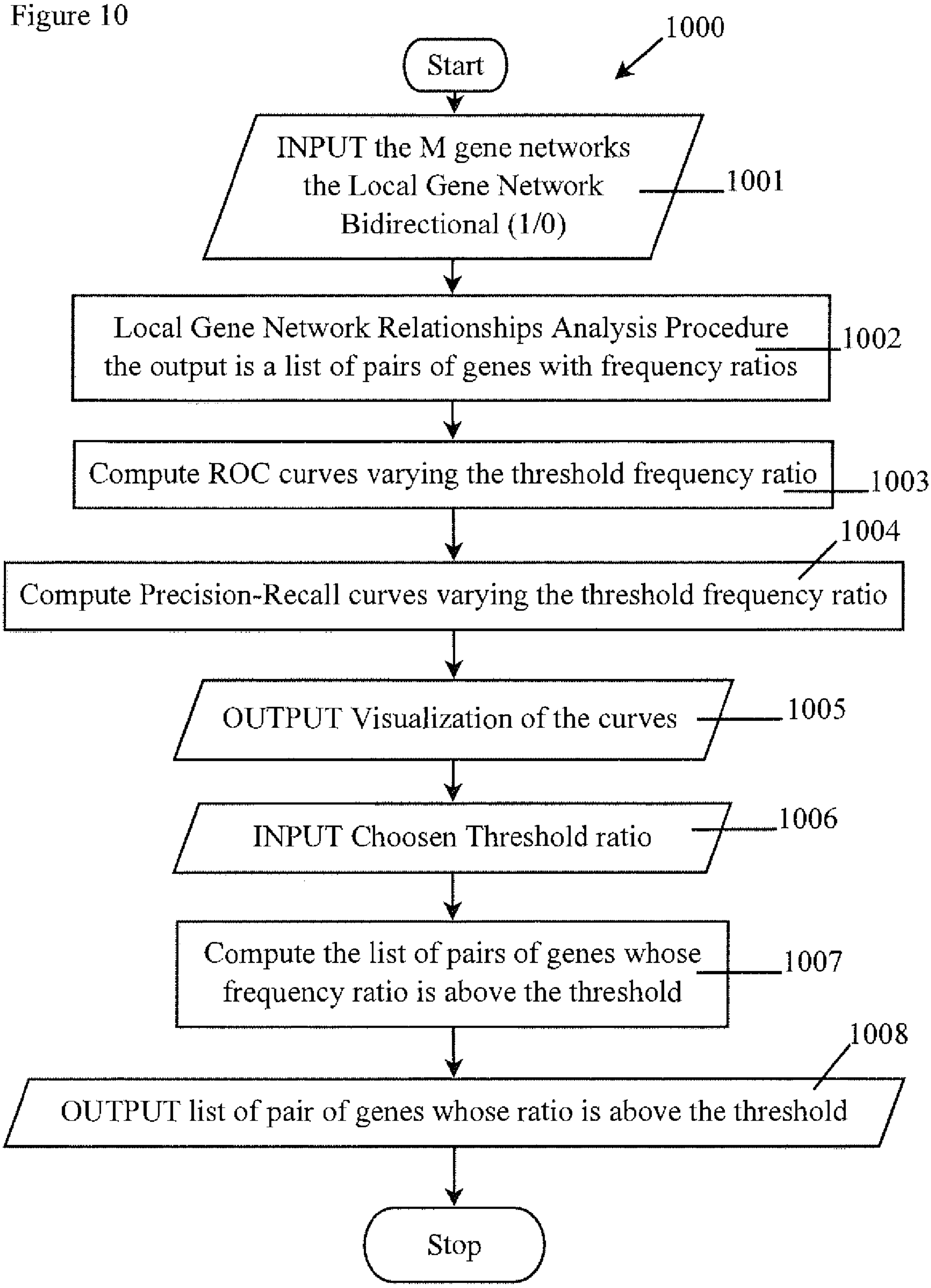
1000
Start
INPUT the M gene networks the Local Gene Network Bidirectional (1/0)
1001
Local Gene Network Relationships Analysis Procedure the output is a list of pairs of genes with frequency ratios
1002
Compute ROC curves varying the threshold frequency ratio
1003
1004
Compute Precision-Recall curves varying the threshold frequency ratio
OUTPUT Visualization of the curves
1005
INPUT Choosen Threshold ratio
1006
Compute the list of pairs of genes whose frequency ratio is above the threshold
1007
1008
OUTPUT list of pair of genes whose ratio is above the threshold
Stop

Figure 11

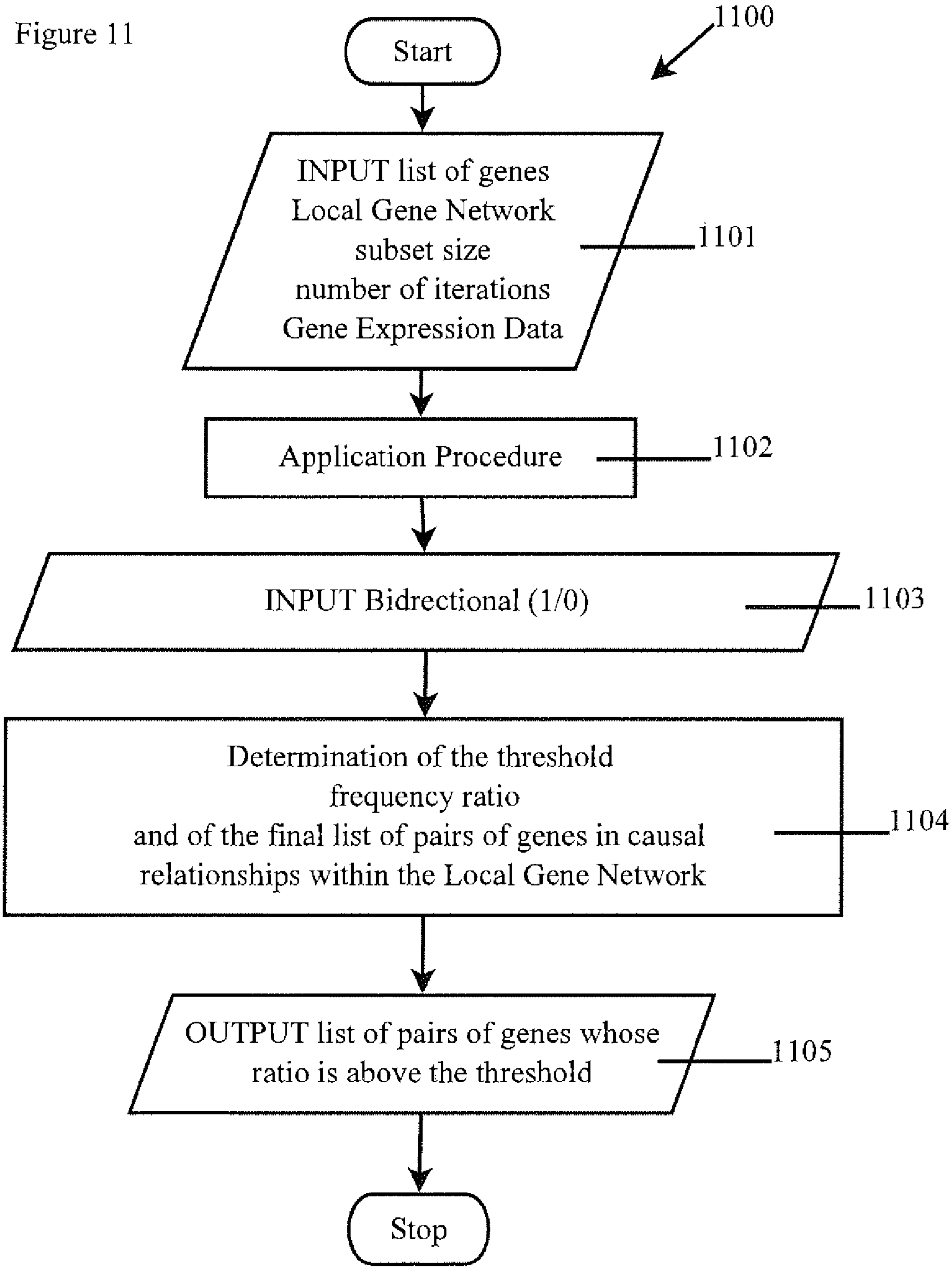
1100
Start
INPUT list of genes
Local Gene Network
subset size
number of iterations
Gene Expression Data
1101
Application Procedure
1102
INPUT Bidirectional (1/0)
1103
Determination of the threshold
frequency ratio
and of the final list of pairs of genes in causal
relationships within the Local Gene Network
1104
OUTPUT list of pairs of genes whose
ratio is above the threshold
1105
Stop

Figure 12

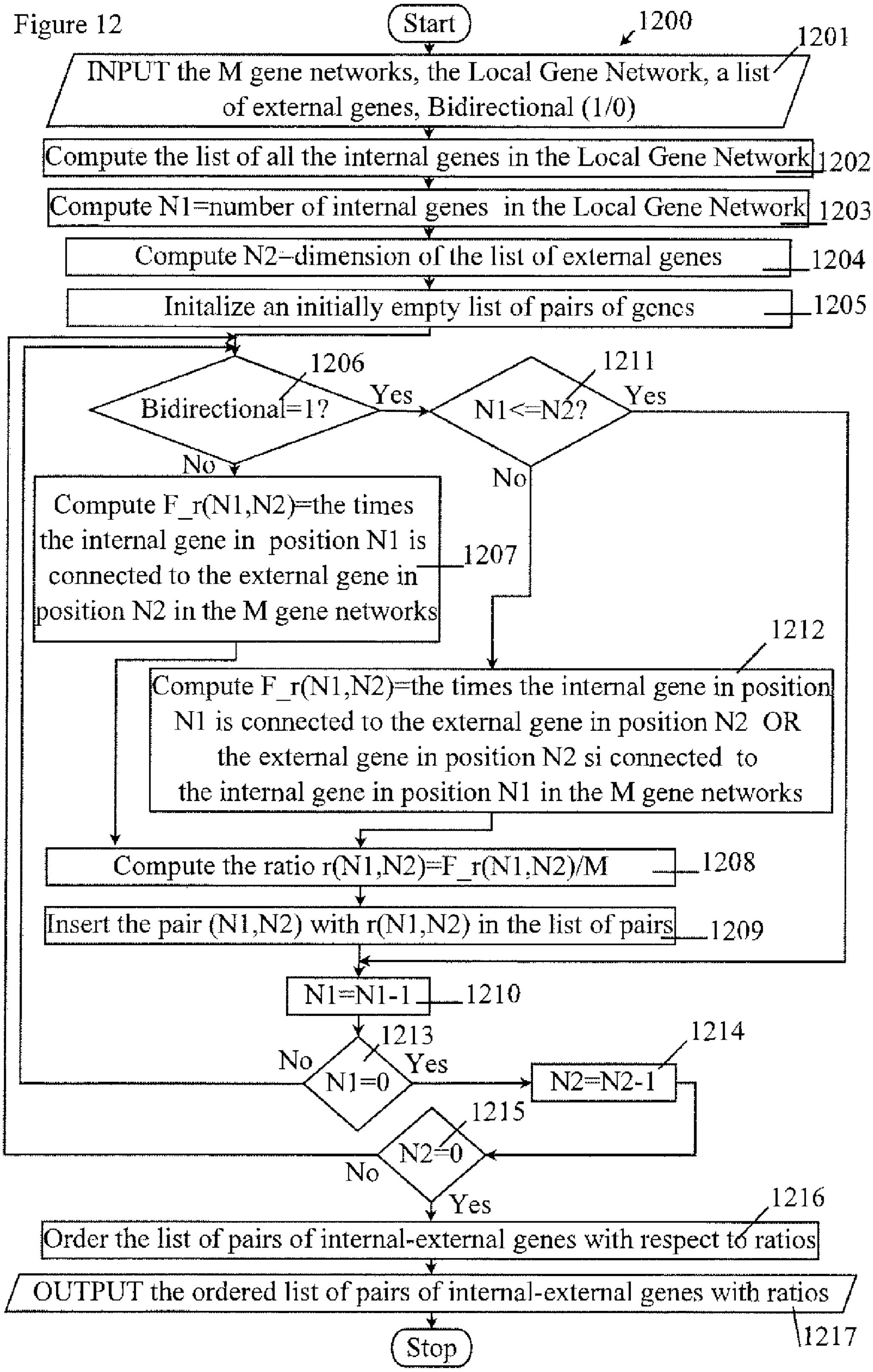
Start
1200
1201
INPUT the M gene networks, the Local Gene Network, a list of external genes, Bidirectional (1/0)
Compute the list of all the internal genes in the Local Gene Network
1202
Compute N1=number of internal genes in the Local Gene Network
1203
Compute N2=dimension of the list of external genes
1204
Initalize an initially empty list of pairs of genes
1205
1206
Bidirectional=1?
Yes
No
1211
N1<=N2?
Yes
No
Compute F_r(N1,N2)=the times the internal gene in position N1 is connected to the external gene in position N2 in the M gene networks
1207
1212
Compute F_r(N1,N2)=the times the internal gene in position N1 is connected to the external gene in position N2 OR the external gene in position N2 si connected to the internal gene in position N1 in the M gene networks
Compute the ratio r(N1,N2)=F_r(N1,N2)/M
1208
Insert the pair (N1,N2) with r(N1,N2) in the list of pairs
1209
N1=N1-1
1210
1213
No
N1=0
Yes
1214
N2=N2-1
1215
N2=0
No
Yes
1216
Order the list of pairs of internal-external genes with respect to ratios
OUTPUT the ordered list of pairs of internal-external genes with ratios
1217
Stop

Figure 13

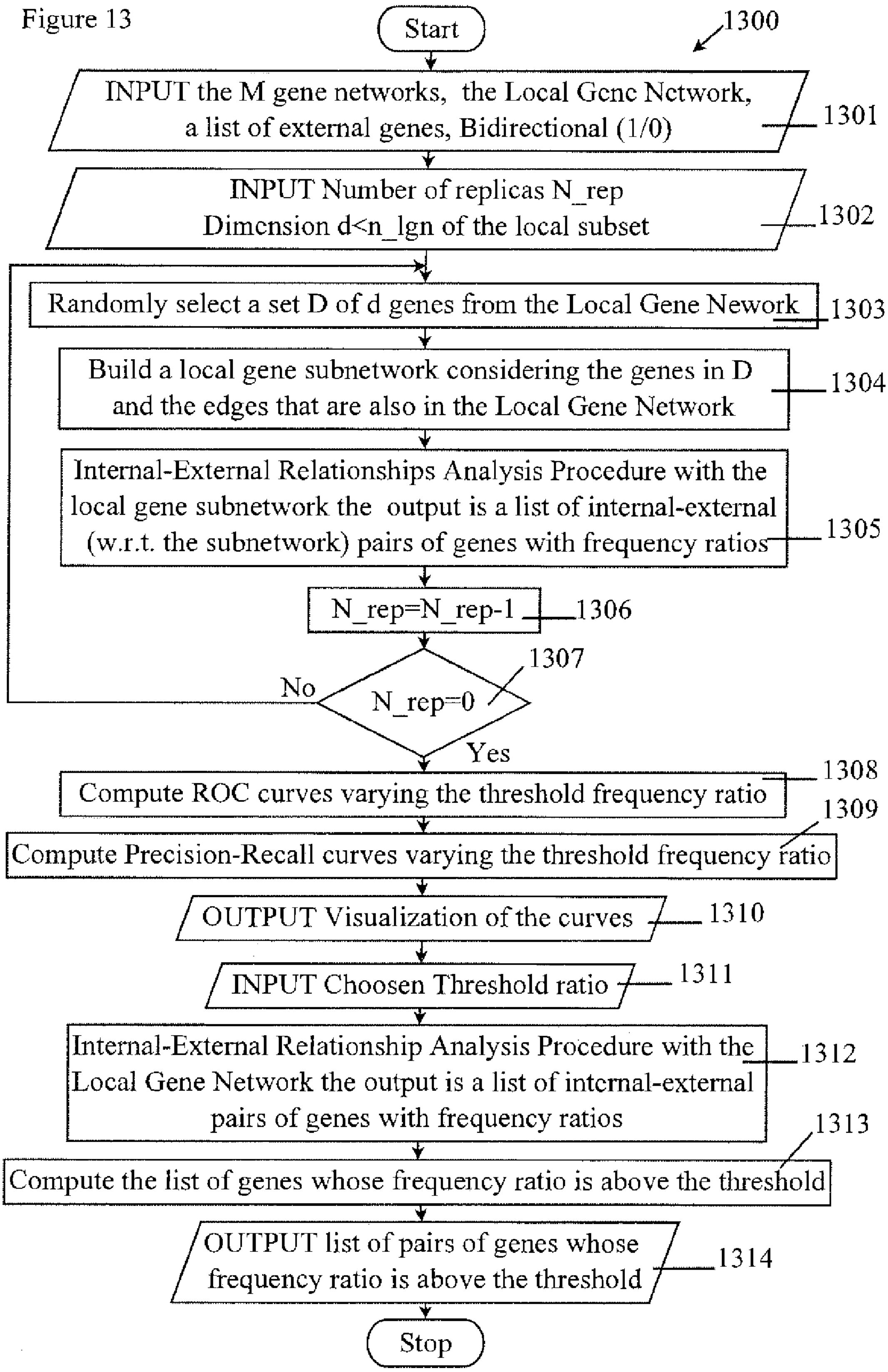

Start
1300
INPUT the M gene networks, the Local Gene Network, a list of external genes, Bidirectional (1/0)
1301
INPUT Number of replicas N_rep
Dimension d<n_lgn of the local subset
1302
Randomly select a set D of d genes from the Local Gene Nework
1303
Build a local gene subnetwork considering the genes in D and the edges that are also in the Local Gene Network
1304
Internal-External Relationships Analysis Procedure with the local gene subnetwork the output is a list of internal-external (w.r.t. the subnetwork) pairs of genes with frequency ratios
1305
N_rep=N_rep-1
1306
1307
No
N_rep=0
Yes
Compute ROC curves varying the threshold frequency ratio
1308
1309
Compute Precision-Recall curves varying the threshold frequency ratio
OUTPUT Visualization of the curves
1310
INPUT Choosen Threshold ratio
1311
Internal-External Relationship Analysis Procedure with the Local Gene Network the output is a list of internal-external pairs of genes with frequency ratios
1312
1313
Compute the list of genes whose frequency ratio is above the threshold
OUTPUT list of pairs of genes whose frequency ratio is above the threshold
1314
Stop

Figure 14

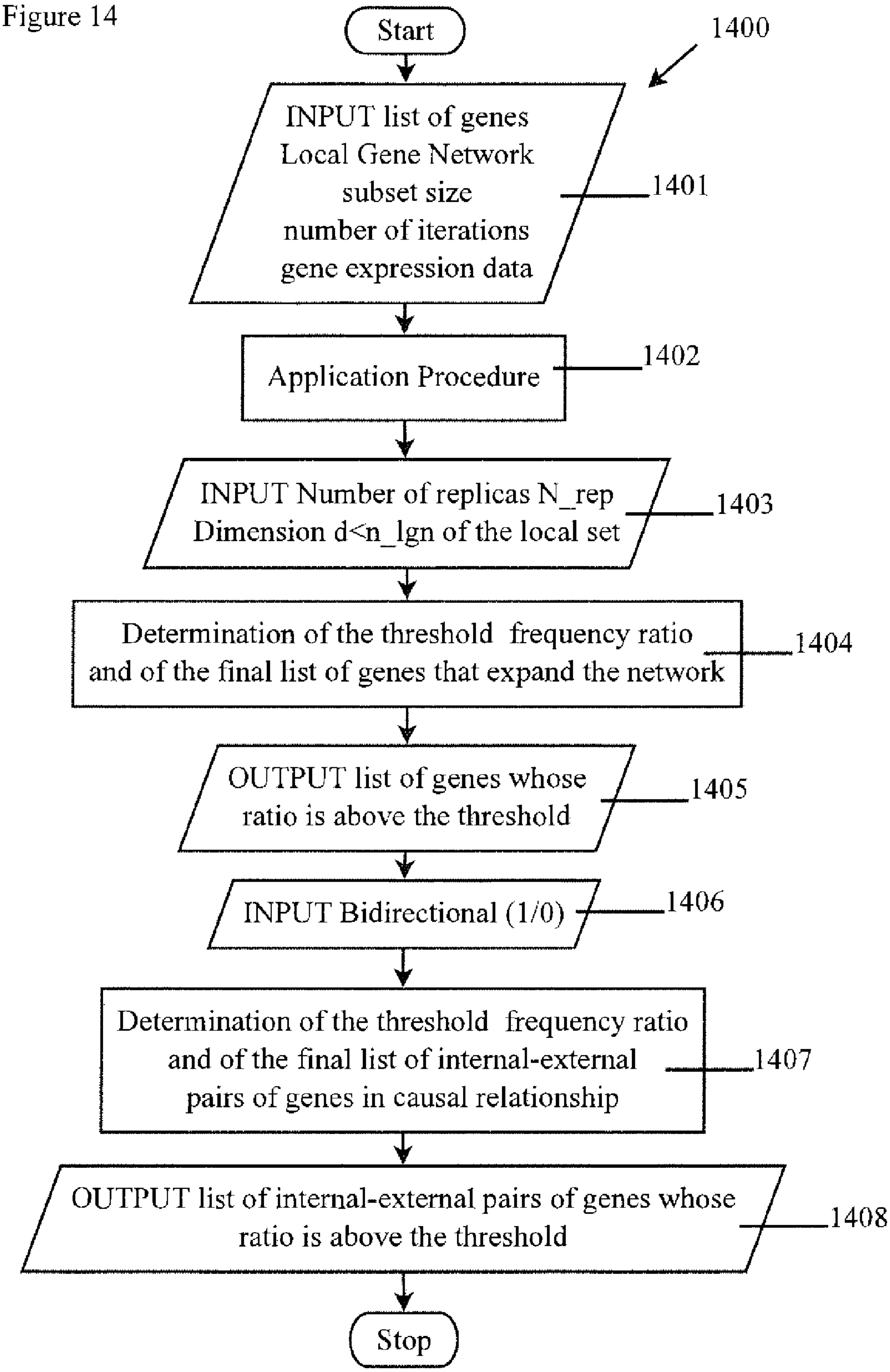
Start
1400
INPUT list of genes
Local Gene Network
subset size
number of iterations
gene expression data
1401
Application Procedure
1402
INPUT Number of replicas N_rep
Dimension d<n_lgn of the local set
1403
Determination of the threshold frequency ratio
and of the final list of genes that expand the network
1404
OUTPUT list of genes whose
ratio is above the threshold
1405
INPUT Bidirectional (1/0)
1406
Determination of the threshold frequency ratio
and of the final list of internal-external
pairs of genes in causal relationship
1407
OUTPUT list of internal-external pairs of genes whose
ratio is above the threshold
1408
Stop

Figure 15

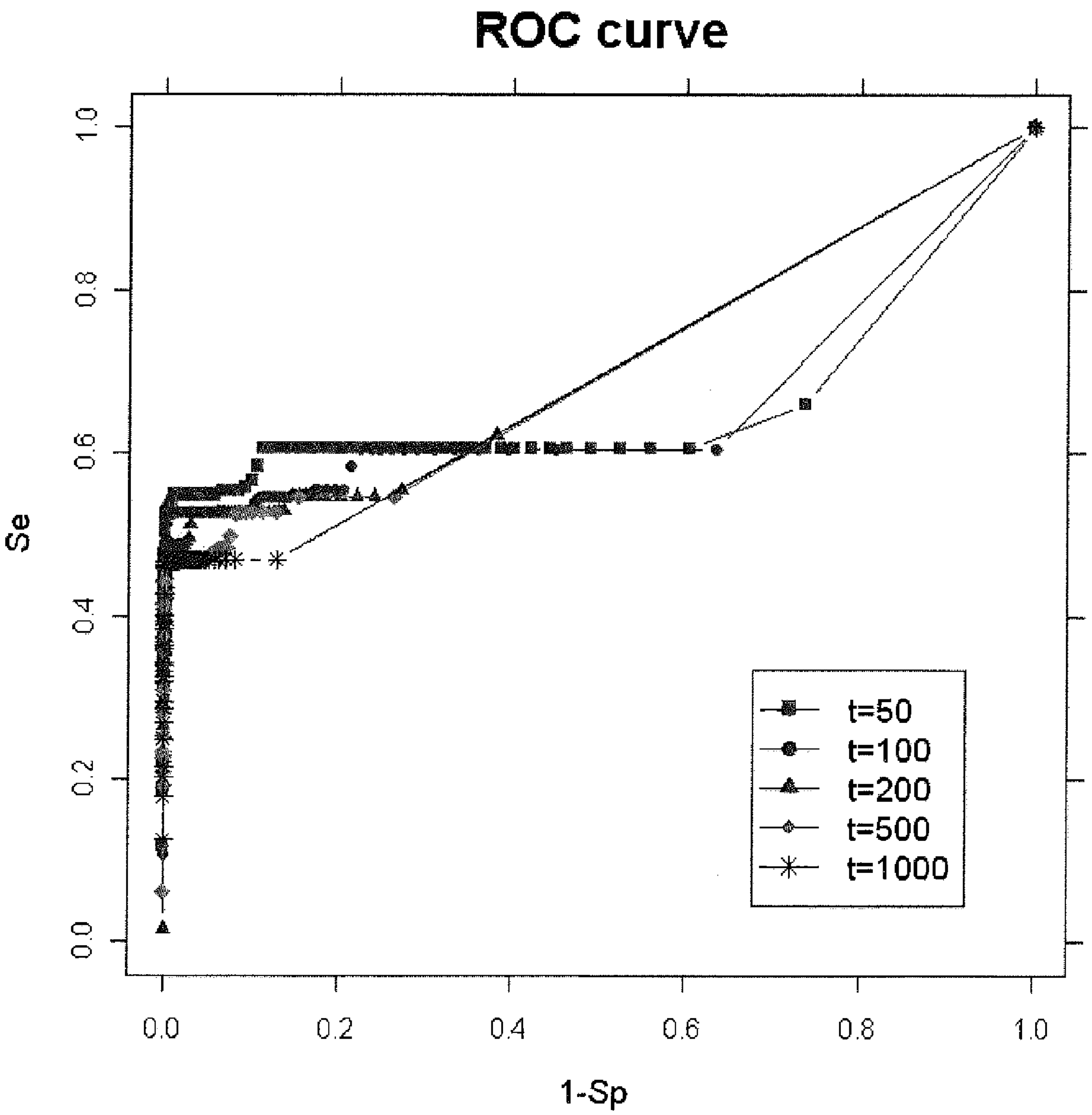

Figure 16

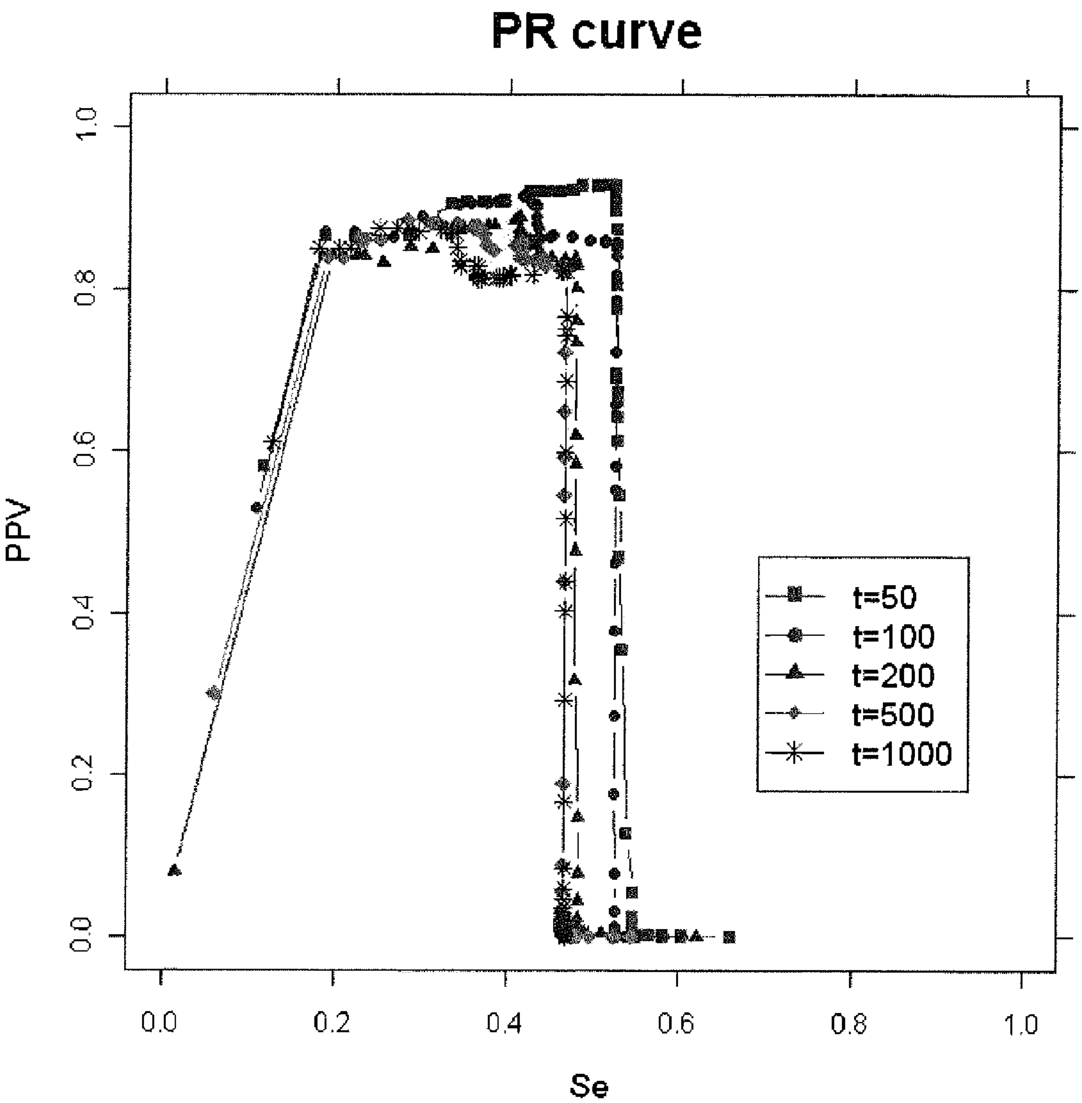

Figure 17

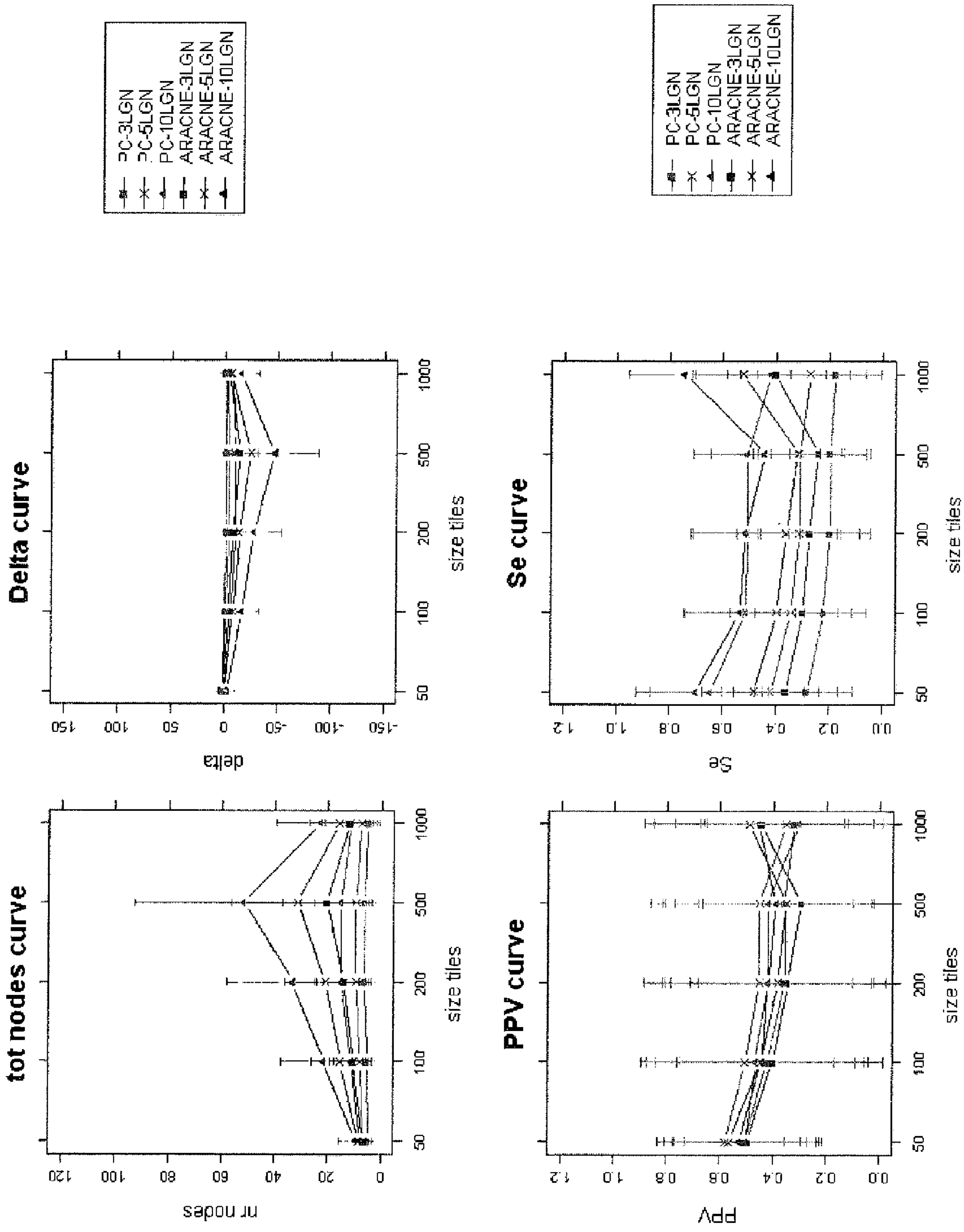

tot nodes curve
Delta curve
PPV curve
Se curve
nr nodes
delta
PPV
Se
size tiles
PC-3LGN
PC-5LGN
PC-10LGN
ARACNE-3LGN
ARACNE-5LGN
ARACNE-10LGN

Figure 18

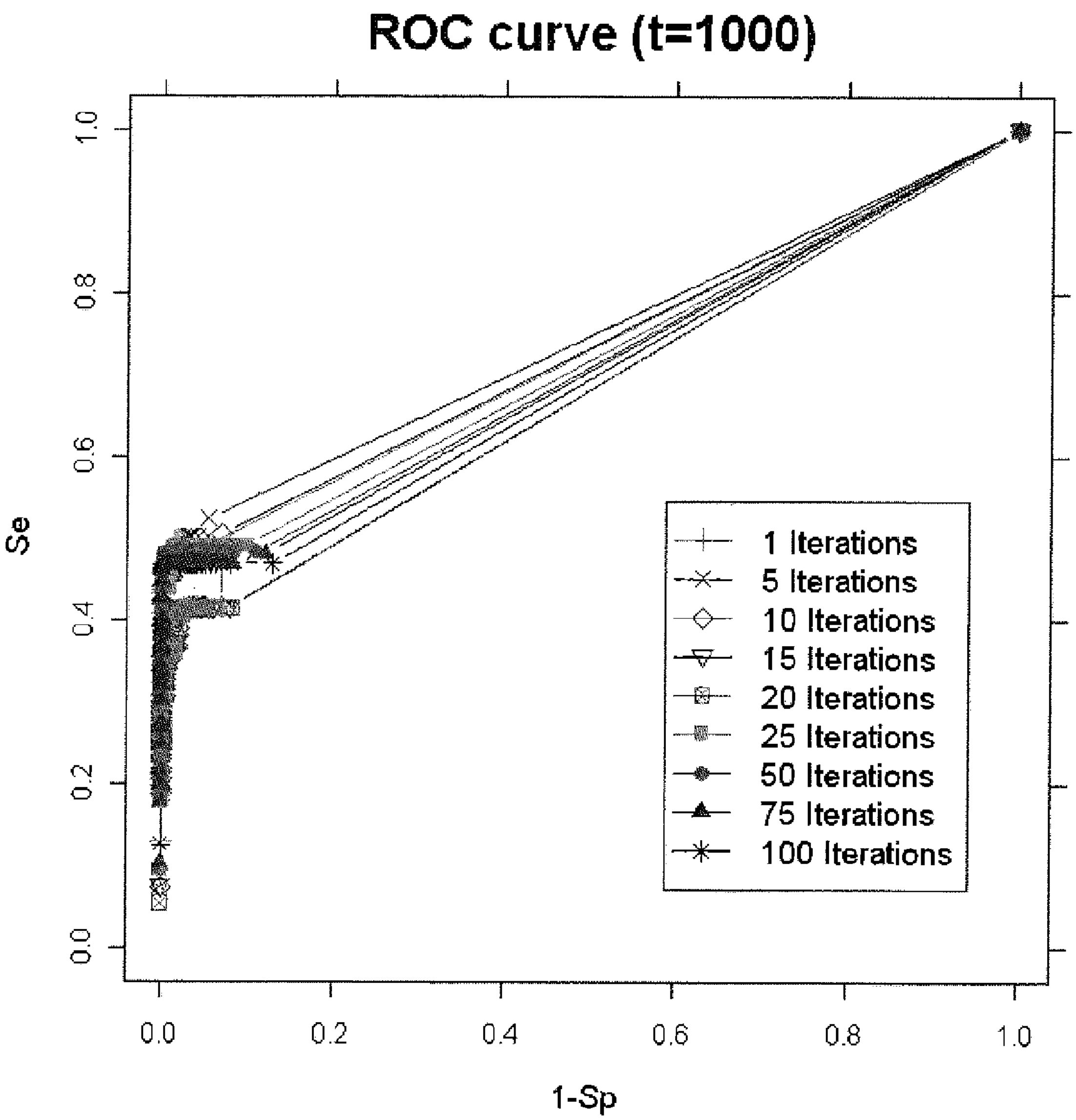
ROC curve (t=1000)
Se
1-Sp
0.0
0.2
0.4
0.6
0.8
1.0
1 Iterations
5 Iterations
10 Iterations
15 Iterations
20 Iterations
25 Iterations
50 Iterations
75 Iterations
100 Iterations

Figure 19

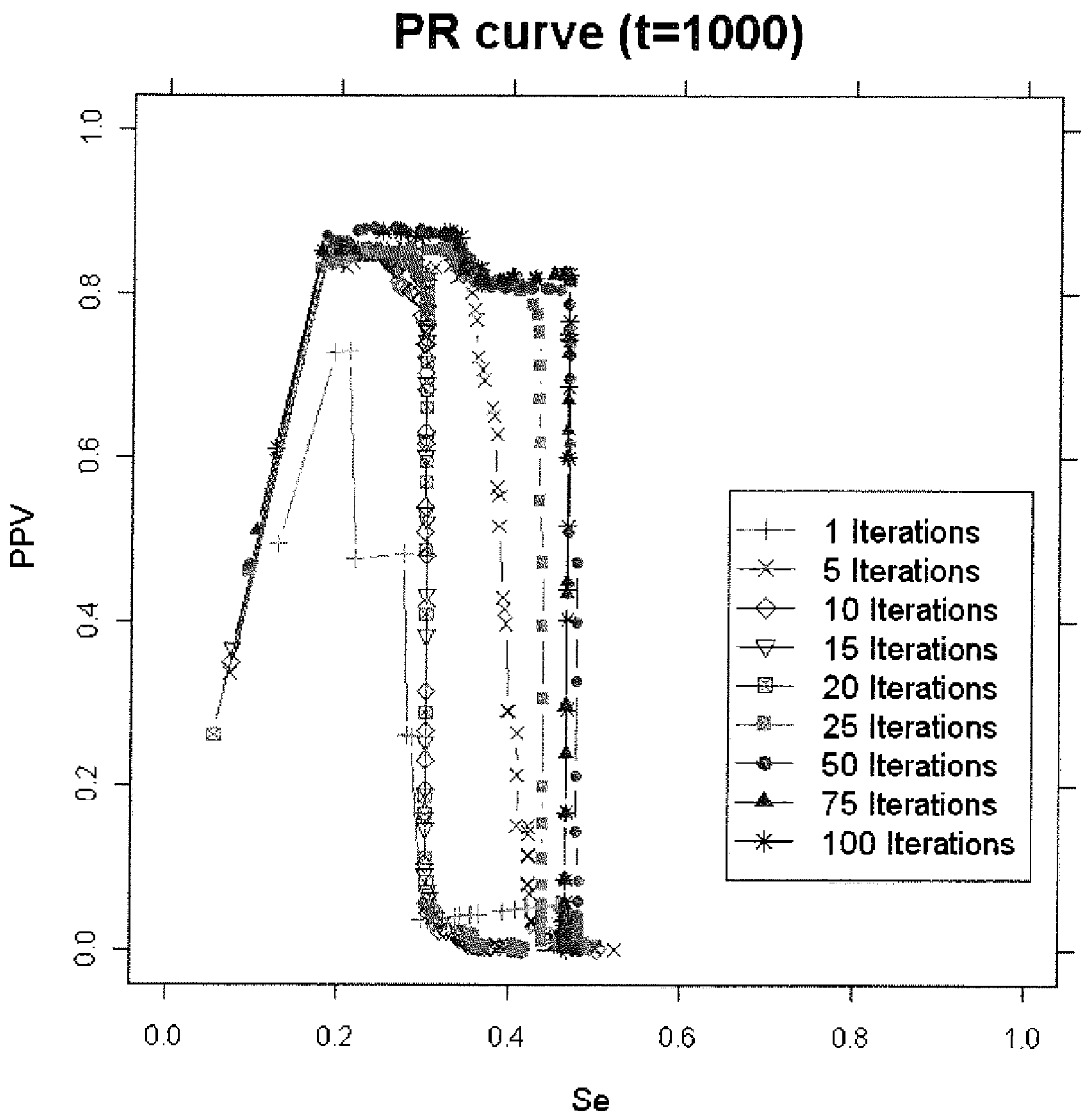
PR curve (t=1000)
PPV
Se
0.0
0.2
0.4
0.6
0.8
1.0
1 Iterations
5 Iterations
10 Iterations
15 Iterations
20 Iterations
25 Iterations
50 Iterations
75 Iterations
100 Iterations

Europäisches Patentamt
European Patent Office
Office européen des brevets

# EUROPEAN SEARCH REPORT

Application Number
EP 13 15 1728

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/103608 A2 (UNIV RAMOT [IL]; SHAMIR RON [IL]; TANAY AMOS [IL]) 27 December 2002 (2002-12-27) * pp. 4,15,26 * ----- | 1-15 | INV. G06F19/12<br>ADD. G06F19/20 |
| A | DANIEL MARBACH ET AL: "Wisdom of crowds for robust gene network inference", NATURE METHODS, vol. 9, no. 8, 15 July 2012 (2012-07-15), pages 796-804, XP055066820, ISSN: 1548-7091, DOI: 10.1038/nmeth.2016 * the whole document * ----- | 1-15 | |
| A | M. J. HERRGARD: "Reconciling Gene Expression Data With Known Genome-Scale Regulatory Network Structures", GENOME RESEARCH, vol. 13, no. 11, 1 November 2003 (2003-11-01), pages 2423-2434, XP055066721, ISSN: 1088-9051, DOI: 10.1101/gr.1330003 * the whole document * ----- | 1-15 | TECHNICAL FIELDS SEARCHED (IPC)<br>G06F |
| A | XIAOGANG WU ET AL: "Network expansion and pathway enrichment analysis towards biologically significant findings from microarrays.", JOURNAL OF INTEGRATIVE BIOINFORMATICS (JIB), vol. 9, no. 2, 1 January 2012 (2012-01-01), pages 213-213, XP055066723, ISSN: 1613-4516, DOI: 10.2390/biecoll-jib-2012-213 * the whole document * ----- | 1-15 | |

-/--

The present search report has been drawn up for all claims

1

| Place of search | Date of completion of the search | Examiner |
|---|---|---|
| Munich | 18 June 2013 | Schmitt, Constanze |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 15 1728

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | D'HAESELEER P ET AL: "Genetic network inference: from co-expression clustering to reverse engineering", BIOINFORMATICS, OXFORD UNIVERSITY PRESS, SURREY, GB, vol. 16, no. 8, 1 August 2000 (2000-08-01), pages 707-726, XP002205151, ISSN: 1367-4803, DOI: 10.1093/BIOINFORMATICS/16.8.707 * the whole document * ----- | 1-15 | |
| A | KYODA K M ET AL: "A GENE NETWORK INFERENCE METHOD FROM CONTINUOUS-VALUE GENE EXPRESSION DATA OF WILD-TYPE AND MUTANTS", PROCEEDINGS GENOME INFORMATICS WORKSHOP, UNIVERSITY ACADEMY PRESS, TOKYO, JP, vol. 11, 18 December 2000 (2000-12-18), pages 196-204, XP002909075, ISSN: 0919-9454 * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| Munich | 18 June 2013 | Schmitt, Constanze |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 15 1728

This annex lists the patent family members relating to the patent documents cited in the above-mentioned European search report.
The members are as contained in the European Patent Office EDP file on
The European Patent Office is in no way liable for these particulars which are merely given for the purpose of information.

18-06-2013

| Patent document cited in search report | Publication date | Patent family member(s) | Publication date |
|---|---|---|---|
| WO 02103608 A2 | 27-12-2002 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20090063376 A


### Non-patent literature cited in the description


- **P., GLYMOUR, C. ; SCHEINES, R.** Causation, Prediction and Search. Springer Verlag, 1993
- **SPIRTES, P. ; GLYMOUR, C. ; SCHEINES, R.** An algorithm for fast recovery of sparse causal graphs. *Social Science Computer Review,* 1991, vol. 9, 62-72
- **T.S. ; PEARL, J.** Equivalence and synthesis of causal models. *Proceedings of the Conference on Uncertainty in Artificial Intelligence,* 1990, 220-227
- **F.C. WIMBERLY ; T. HEIMAN ; J. RAMSEY ; C. GLYMOUR.** Experiments on the accuracy of algorithms for inferring the structure of genetic regulatory networks from microarray expression levels. *Proc. IJCAI 2003 Bioinformatics Workshop,* 2003
- **WANG M. ; BENEDITO V.A. ; ZHAO P.X. ; UDVARDI M.** Inferring large-scale gene regulatory networks using a low-order constraint-based algorithm. *Molecular BioSystems,* 2010, vol. 6, 988-998
- **ZHANG XJ ; ZHAO XM ; HE K ; LU L ; CAO YW ; LIU JD ; HAO JK ; LIU ZP ; CHEN LN.** Inferring gene regulatory networks from gene expression data by path consistency algorithm based on conditional mutual information. *Bioinformatics,* 2012, vol. 28, 98-104
- **KALISCH M. ; BUHLMANN P.** Estimating High-Dimensional Directed Acyclic Graphs with the PC-Algorithm. *Journal of Machine Learning Research,* 2007, vol. 8, 613-636
- **M. TAN ; M. ALSHALALFA ; R. ALHAJJ ; F. POLAT.** Combining Multiple Types of Biological Data in Constraint-Based Learning of Gene Regulatory Networks. *Proc. IEEE Fifth Symp. Computational Intelligence in Bioinformatics and Computational Biology,* 2008, 91-98
- **ANDREW P. HODGES ; DONGJUAN DAI ; ZUOSHUANG XIANG ; PETER WOOLF ; CHUANWU XI ; YONGQUN HE.** Bayesian Network Expansion Identifies New ROS and Biofilm Regulators. *PLoS ONE,* March 2010, vol. 5 (3), e9513, www.plosone.org
- **MARBACH,D. ; SCHAFFTER,T. ; MATTIUSSI,C. ; FLOREANO,D.** Generating realistic in silico gene networks for performance assessment of reverse engineering methods. *J Comput Biol.,* February 2009, vol. 16 (2), 229-39
- **WANG, C. ; MARSHALL, A. ; ZHANG, D. ; WILSON, Z.A.** ANAP: An Integrated Knowledge Base for Arabidopsis Protein Interaction Network Analysis. *Plant physiology,* 2012, vol. 158 (4), 1523-33
- **SANCHEZ-CORRALES,Y.E. ; ALVAREZ-BUYLLA,E.R. ; MENDOZA,L.** The Arabidopsis thaliana flower organ specification gene regulatory network determines a robust differentiation process. *Journal of theoretical biology,* 2010, vol. 264 (3), 971-83